# EUROPEAN PATENT APPLICATION

(11) **EP 3 670 656 A1**
(43) Date of publication of application: **24.06.2020**
(21) Application number: 18846527.2
(22) Date of filing: 16.08.2018
(51) Int. Cl.: C12N 15/09, C12M 1/00, C12N 1/15, C12N 1/19, C12N 5/071, C12N 5/10, C12N 15/12, C12Q 1/02, G01N 21/64

(54) **METHOD FOR QUANTIFYING ODORS, CELLS USED IN SAME, AND METHOD FOR PRODUCING SAID CELLS**

(30) Priority: 17.08.2017 JP 2017157492
(71) Applicant: Komi Hakko Corporation, Osaka-shi, Osaka 550-0002 (JP)
(72) Inventor: KURODA, Shun'ichi, Osaka-shi Osaka 532-0006 (JP)
(74) Representative: Appleyard Lees IP LLP
(86) International application number: PCT/JP2018/030453
(87) International publication number: WO 2019/035476

(57) **Abstract**

The purpose of the present invention is to provide a method for constructing a device for causing a plurality of receptors to be expressed in respectively different eukaryotic cells upon nucleic acids within through-holes, housing a plurality of eukaryotic cells within the through-holes of the device and causing the receptors to be expressed therein, comprehensively measuring the degree of activation of the plurality of receptors by a test substance, and evaluating the degree of activation as a specific numerical value. Provided is an array which includes a substrate and a hydrophobic coating film upon the substrate, the hydrophobic coating film having a plurality of through-holes, and a nucleic acid containing a gene which codes for a prescribed receptor being provided in the interior of each through-hole in a manner touching the substrate.

## Description

### TECHNICAL FIELD

The present invention relates to a method for quantifying odors, and cells used in the same.

### BACKGROUND ART

Receptors cause signal transduction by action with an extracellular substance (ligand). There exist various types of receptor depending on ligands and functions; however, responsiveness between a receptor and a ligand has not been fully understood. In addition, proteins having a structure similar to that of receptors, whose function has been unknown, have been found by gene analysis in recent years, and some are called orphan receptors. In order to examine a relationship between a receptor and a ligand, it is expected to establish a receptor array in which prescribed receptors having different genetic information are arranged one by one, and a measurement technique for measuring the responsiveness of receptors by allowing a prescribed ligand to simultaneously act on the array.

When focusing on various receptors, particularly olfactory receptors, animals recognize external odor information by an organ called the nose and discriminate it. The discrimination capability is very high, and trained animals (including humans) can also discriminate odor substances which cannot be detected by a detection apparatus such as gas chromatography. If a highly developed mechanism of recognizing odors possessed by such trained animals is imitated and this can be made into a detection device, an odor detection device can be created which can discriminate various odors with high sensitivity, which cannot be achieved by a detection device using a detection apparatus such as existing gas chromatography.

Olfactory receptors are a type of 7-transmembrane receptor expressed in olfactory sensory neurons, and interact with G proteins in the intracellular region of olfactory receptors. Olfactory receptors are activated by stimulation of an odor molecule from the outside of cells, and Gaolf protein, a type of G protein, is then activated in cells. This Gaolf activates adenylate cyclase and accumulates cAMP in olfactory sensory neurons using ATP as a raw material. Then cAMP is bound to Cyclic Nucleotide Gated ion-channel (CNG) on the cell membrane of an olfactory sensory neuron to open the channel, and calcium ion flows into cells through CNG. Consequently, the concentration of calcium ion in the cells increases.

Patent Document 1: Pamphlet of PCT International Publication No. WO03/100057

### DISCLOSURE OF THE INVENTION

### Problems to be Solved by the Invention

When receptors are used as a device to examine action with an extracellular ligand, a technique for constructing several hundred types of receptor into a device so that the degree of activation of receptors can be measured has not been conventionally established.

When a receptor is an olfactory receptor, for example, because an odor molecule is bound to a protein called an olfactory receptor expressed on the surface of an olfactory sensory neuron in the nasal cavity, signal transduction is caused in the olfactory sensory neuron, and odor information is transmitted to the brain. It is thought that there exist about 400 types of olfactory receptor in humans and about 1100 types in mice by genome analysis, and it is understood that a group of olfactory receptors is activated to different degrees by a specific odor molecule and these stimulations are transmitted to the brain and combined; accordingly, various odors are discriminated in the brain.

In order to create an odor detection device which can discriminate various odors with high sensitivity as described above, however, it is necessary to construct a system in which all of the above approximately 400 types (or 1100 types) of olfactory receptor are expressed on each of a plurality of cultured cells other than olfactory sensory neurons with the function actually shown, and the degree of activation of each olfactory receptor by odor stimulation can be specifically compared.

However, when the receptor is an olfactory receptor, cells expressing the olfactory receptor at a level suitable to measure the degree of activation of the receptor (i.e. presentation to the cell surface, a sufficient expression level, and maintenance of an efficient conjugated state with intracellular signal transduction proteins) have not been established.

A vector including a foreign gene to express such an olfactory receptor has not also been established.

As for a measurement system, a method for measuring the degree of activation of receptors with high accuracy has not also been established because the above level is not constant on a cell basis even in cells expressing an identical receptor. Furthermore, a measurement technique for comprehensively measuring the degree of activation of several hundred types of receptor in a whole device has not also been established.

Patent Document 1 describes a method for measuring the activation of an expressed olfactory receptor by an odor molecule, but does not describe a method for uniformly evaluating the degree of activation of a cell group with a plurality of expressed olfactory receptors, by each odor stimulation.

A method for forming a target odor based on the measurement values of the degree of activation using cells expressing an olfactory receptor has not also been established.

A method for modifying an odor into a target odor based on the measurement values of the degree of activation using cells expressing an olfactory receptor has not also been established.

Therefore, an object of the present invention is to provide a receptor array in which a prescribed receptor is exhaustively arranged, and a measurement system for comprehensively measuring the degree of activation of each receptor by allowing a prescribed ligand to simultaneously act on the array. An object thereof is also to provide a method for evaluating the degree of activation (hereinafter can be referred to as activation degree) of each receptor by an extracellular ligand as a specific numerical value by constructing a system in which a plurality of receptors are each expressed on different cells. An object thereof is also to provide a method for forming a target odor based on the activation degree measured when the receptor is an olfactory receptor, and moreover a method for modifying an odor into a target odor.

### Means for Solving the Problems

As a result of repeated diligent investigations to solve the above problems by the present inventors, the present invention was completed, and widely includes, for example, the invention of aspects described below.
(1) An array, including: a substrate, and a hydrophobic coating film on the substrate, wherein the hydrophobic coating film has a plurality of through-holes, and a nucleic acid including a gene which codes for a prescribed receptor is provided in contact with the substrate in the interior of the through-holes.
(2) The array according to (1), wherein nucleic acids including genes which code for the prescribed receptors different from each other are provided in each of the through-holes.
(3) The array according to (1) or (2), wherein the average value of the cross-sectional area of the plurality of through-holes is 0.125 mm² or more and 0.283 mm² or less.
(4) The array according to (3), wherein 200 or more of the through-holes are provided in the substrate and also the average pitch between the through-holes is 0.6 mm or more and 0.8 mm or less.
(5) The array according to (1) to (4), wherein the hydrophobic coating film has a contact angle with water at 23°C of 70° or more.
(6) The array according to (1) to (5), wherein the prescribed receptor includes a G protein-coupled receptor.
(7) The array according to (6), wherein the G protein-coupled receptor includes an olfactory receptor.
(8) The array according to (1) to (7), wherein a plurality of eukaryotic cells are further held in one of the through-holes.
(9) Eukaryotic cells, which express CNGA2 and GNAL or mutants thereof as foreign genes.
(10) The cells according to (9), which further express CNGA4 and/or CNGBlb or mutants thereof as foreign genes.
(11) The cells according to (9) or (10), which further express a gene which codes for a protein which can transfer an olfactory receptor expressed in the cells from the endoplasmic reticulum membrane to the cell membrane to raise the efficiency of presentation of the olfactory receptor to the surface of the cells.
(12) At least one vector, which is selected from a group consisting of:
   (1) Vector A having genes which code for CNGA2 and GNAL or mutants thereof,
   (2) Vector B having genes which code for CNGA4 and/or CNGBlb or mutants thereof, and
   (3) Vector C having a gene which codes for a protein which can transfer an olfactory receptor expressed in cells from the endoplasmic reticulum membrane to the cell membrane to raise the efficiency of presentation of the olfactory receptor to the surface of the cells.
(13) Eukaryotic cells, into which at least one vector according to (12) is incorporated.
(14) Eukaryotic cells having a region to incorporate a certain number of genes which code for an olfactory receptor derived from a mammal onto the chromosomes of cells according to any of (9) to (11) or (13).
(15) Eukaryotic cells, in which a human-derived olfactory receptor gene is incorporated into the region according to (14) .
(16) The array according to (8), wherein the eukaryotic cells are eukaryotic cells according to (9) to (11) or (13) to (15) .
(17) A method for calculating the activation degree of a prescribed receptor by a test substance, the method including:
   (1) a step of measuring, when the test substance is brought into contact with a plurality of eukaryotic cells expressing the receptor, the amount of ions taken into each of the cells,
   (2) a step of measuring, when the same cells as used in the measurement in step 1 are depolarized, the amount of ions in each of the cells, and
   (3) a step of calculating a ratio of a numerical value measured in step 1 and a numerical value measured in step 2.
(18) The method according to (17), wherein, in step 1 and step 2, a means for measuring the amount of ions taken into the cells is a means using an ion-measuring pigment or a means using an ion-binding fluorescent protein.
(19) The method according to (17) or (18), wherein the depolarization step according to step 2 is a step of applying a potassium compound or an ionophore to the eukaryotic cells.
(20) A method for calculating the activation degree of a receptor by a test substance, the method including: a step of measuring, when the test substance is brought into contact with a plurality of eukaryotic cells expressing the receptor, the amount of substance taken into each of the cells, wherein, when the average value of fluorescence in a first prescribed time before the contact is Fbase and the greatest value of fluorescence in a second prescribed time after the contact is Fmax, Fmax/Fbase is calculated and used as an activation degree.
(21) The method according to (20), wherein, when the standard deviation of fluorescence in the first prescribed time is used as SD and the Fmax is not less than Fbase + 5 SD, the Fmax is used as an activation degree, and when the Fmax is less than Fbase + 5 SD, the Fmax is not used as an activation degree.
(22) The method according to (20) or (21), wherein after the measurement step about a certain test substance, a positive control substance is not brought into contact with the eukaryotic cells, and a next test substance is brought into contact with eukaryotic cells.
(23) The method according to (22), wherein contact with the next test substance is carried out at a time when a fluorescence value from the certain test substance is not less than Fbase + 5 SD.
(24) The method according to (17) to (23), wherein receptor expression of the eukaryotic cells is transient expression.
(25) The method according to (24), wherein the receptor is an olfactory receptor.
(26) A method for measuring the activation degree of a receptor by a test substance, the method including: a step of allowing an ion-measuring pigment or an ion-binding fluorescent protein to act on the array according to (16), a step of bringing the test substance into contact with the array, a step of simultaneously acquiring a region including all the through-holes as image data, a step of individually recognizing the eukaryotic cells and obtaining fluorescence in each of the eukaryotic cells as a numerical value, and a step of calculating an activation degree by arithmetic processing of the numerical value.
(27) The method according to (26), wherein the arithmetic processing is processing by which, when the average value of fluorescence in a first prescribed time before the contact is Fbase and the greatest value of fluorescence in a second prescribed time after the contact is Fmax, Fmax/Fbase is calculated and used as an activation degree.
(28) The method according to (27), wherein, when the standard deviation of fluorescence in a first prescribed time before the contact is SD and the Fmax is not less than Fbase + 5 SD, the arithmetic processing uses the Fmax as an activation degree, and, when the Fmax is less than Fbase + 5 SD, the arithmetic processing does not use the Fmax as an activation degree.
(29) The method according to (26) to (28), wherein the receptor is a human-derived olfactory receptor.
(30) A method for forming an odor of a target substance, wherein two or more reference substances are combined so that based on the activation degree of each receptor in regard to the two or more reference substances measured using the method of (29), the activation degree of each receptor in regard to the target substance measured using the measurement method is formed.
(31) A method for producing a composition having a formed odor of a target substance, wherein two or more reference substances are combined so that based on the activation degree of each receptor in regard to the two or more reference substances measured using the method of (29), the activation degree of each receptor by the target substance measured using the measurement method is formed.
(32) The method according to (30) or (31), wherein the combination method is an operation of a first data and a second data, wherein the activation degree of each receptor in regard to each of the reference substances is obtained as the first data, and the activation degree of each receptor in regard to the target substance is obtained as the second data.
(33) A method for screening a substance to modify a sample odor into a target odor, the method including a step of selecting a candidate substance as an indicator that the activation degree of each receptor by the sample measured using the measurement method according to (29) is brought close to the activation degree of each receptor in the target odor state measured using the measurement method according to (29) .
(34) The method according to (33), wherein the selection is carried out based on whether or not the activation degree of each of the receptors measured when adding a candidate substance to the sample is brought close to the activation degree in the target odor state.
(35) The method according to (33), wherein the selection includes selection from the reference substances based on existing information about the activation degree of each of the receptors by the reference substances.

### Effects of the Invention

According to the present invention, it is possible to provide a technique for measuring the activation degree of a receptor by a receptor array and a ligand, and an application thereof.

### BRIEF DESCRIPTION OF THE DRAWINGS

Fig. 1 is a plan view which shows an array of the present invention.
Fig. 2 is a plan view which shows a layout of through-holes in the array and a eukaryotic cell array of the present invention.
Fig. 3 is a plan view which shows the eukaryotic cell array of the present invention.
Fig. 4 is a plan view which shows the through-hole and eukaryotic cells of the present invention.
Fig. 5 is a graph which illustrates Fbase and Fmax in fluorescence intensity.
Figs. 6 are graphs which show the response characteristics of olfactory receptors to a test substance.
Fig. 7 is a diagram which illustrates forming an odor using activation degrees.
Fig. 8 is a graph which shows the response characteristics of fluorescence values to test substances.
Fig. 9 is a graph which shows the response characteristics of fluorescence values to test substances.
Fig. 10 is a graph which shows the response characteristics of an olfactory receptor to a test substance.
Figs. 11 are plan views which illustrate a method for processing image data.
Fig. 12 is an image which shows an odor matrix of a target substance.
Figs. 13 are images which illustrate forming an odor using reference substances.

### PREFERRED MODE FOR CARRYING OUT THE INVENTION

The term "absolute" used in the present description is a term used as the opposite of "relative" and means that a value does not easily vary depending on measurement conditions. In addition, the term does not mean that a value does not vary at all even when measurement conditions are changed, but means that a value with sufficient accuracy to achieve the objects of the present invention is obtained even if measurement conditions are changed.

### Array

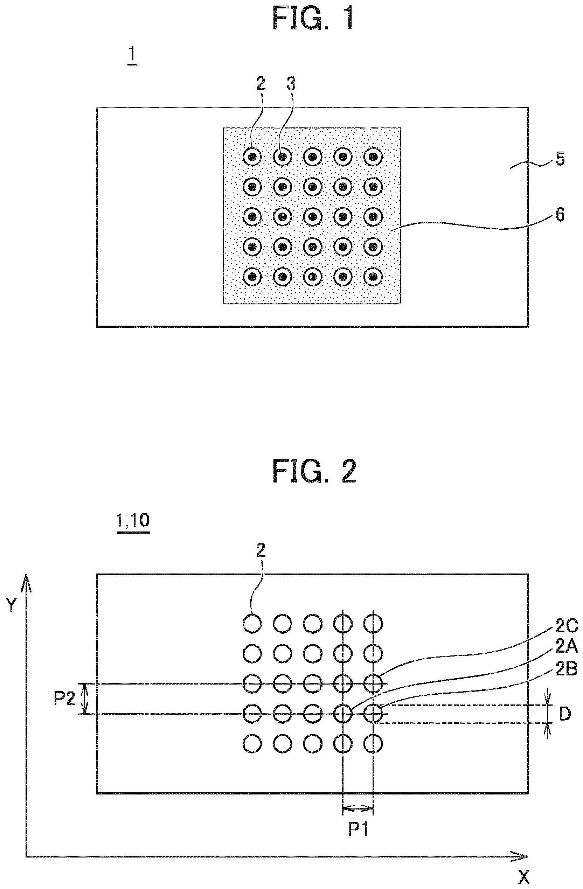

The array of the present invention will be illustrated using Figs. 1 and 2. As shown in Fig. 1, the array 1 of the present invention includes a substrate 5 and a hydrophobic coating film 6 on the substrate 5. A plurality of through-holes 2 are provided in the hydrophobic coating film 6 and the through-holes 2 reach the substrate 5.

As the substrate 5, a material with a strength which can be handled in loading a nucleic acid, loading eukaryotic cells, applying a ligand, and measuring an activation degree by a ligand described below, for example, a material with a Young's modulus at 25°C of 0.01 GPa or more and 1000 GPa or less can be used. A material with 1 or more and 500 GPa or less, which is easily handled with mechanical hands (robot hands) can be preferably used.

It is also preferred that the substrate 5 not show chemical and biological inhibitory effects in loading a nucleic acid, loading eukaryotic cells, and applying a ligand as described below, and examples thereof can include glasses such as fused quartz, borosilicate glass, sodium aluminosilicate glass (including chemically strengthened glass), barium borosilicate glass and soda-lime glass, and various synthetic resins such as ceramics, polycarbonate, polymethyl methacrylate, polystyrene, alicyclic polyolefin and polymethyl pentene. Among these, as synthetic resins, thermoplastic resins, thermosetting resins and radiation curing resins (including examples of ultraviolet rays, electron beams) can be also used as long as attention is paid so that the chemical and biological inhibitory effects described above are not shown, for example as long as attention is paid to the effects of additives. A slide glass using borosilicate glass is given as an example.

The surface of the substrate 5 may be made hydrophilic by surface activation treatment such as plasma treatment, ozone treatment, ion treatment or radiation treatment.

The size of the substrate 5 is not restricted, and typically is preferably a 100 mm angle or less because it is easy to handle by both human and machine. The thickness of the substrate 5 is not also restricted, and typically is preferably above 0 mm and 10 mm or less because it is easy to handle by both human and machine.

The hydrophobic coating film 6 has the effect of moderately repelling eukaryotic cells and easily holding eukaryotic cells in the through-holes 2 in the hydrophobic coating film 6 when loading eukaryotic cells as described below. That is, the hydrophobic coating film 6 has moderate hydrophobicity, and the hydrophobicity can be defined by a contact angle (°) in the planar part other than the through-holes 2 in the hydrophobic coating film 6.

The contact angle of the hydrophobic coating film 6 is desirably 70° or more and 175° or less when using 23°C water. That is, coating films showing a contact angle called water repellency or super water repellency are also included. The contact angle of the hydrophobic coating film 6 of the present invention is typically 75° or more, 80° or more, 90° or more, 100° or more, 110° or more, 120° or more, 130° or more, 135° or more, 140° or more, 145° or more, 150° or more, 155° or more, 160° or more, 165° or more, or 170° or more. The contact angle is also typically 170° or less, 165° or less, 160° or less, 155° or less, 150° or less, 145° or less, 140° or less, 135° or less, 130° or less, 120° or less, 110° or less, 100° or less, 90° or less, or 80° or less. In one embodiment of the present invention, the contact angle of the hydrophobic coating film 6 is 130° or more and 165° or less when using 23°C water.

As specific examples of the hydrophobic coating film 6 having such a contact angle, silicon resins, fluorine resins, and silicon-fluorine resins can be used. Derivatives in which a hydrophobic group is introduced into an aliphatic hydrocarbon such as polyethylene glycol (PEG) or an aromatic hydrocarbon can be also used. Resins obtained by allowing a silicon-based silane coupling agent or a fluorine-based silane coupling agent to act on a thermoplastic resin, a thermosetting resin or a radiation curing resin (including examples of ultraviolet rays, electron beams) can be also used (the action includes copolymerization, surface precipitation and surface coating). As the hydrophobic coating film 6, those which do not show chemical and biological inhibitory effects in loading a nucleic acid, loading eukaryotic cells, and applying a ligand as described below are preferably used.

The thickness of the hydrophobic coating film 6 is not restricted; however, it is desirably 1 mm or more and 200 mm or less because loading a nucleic acid and eukaryotic cells as described below is thus able to be carried out well. The thickness is desirably 10 mm or more and 200 mm or less so that particularly loaded eukaryotic cells are not disconnected due to vibration in the external environment. The thickness is also desirably 25 mm or more and 200 mm or less so that loaded eukaryotic cells are not moved to the outside of the through-holes 2 due to vibration in the external environment and disturbances such as temperature changes. The thickness is typically 30 mm or more, 35 mm or more, 40 mm or more, 45 mm or more, 50 mm or more, 55 mm or more, or 60 mm or more. In addition, the thickness is typically 180 mm or less, 160 mm or less, 140 mm or less, 120 mm or less, 100 mm or less or 80 mm or less.

The hydrophobic coating film 6 may be made by a method for simultaneously making a film of the hydrophobic coating film 6 and through-holes 2 such as offset printing, tampo printing, screen printing or inkjet printing, or through-holes 2 may be made by a lithography method after applying the hydrophobic coating film 6 onto the whole surface. Offset printing and tampo printing are suitable to make a thickness of 1 mm or more and 10 mm or less, and screen printing is suitable to make a thickness of 20 mm or more and 70 mm or less.

A range in which the hydrophobic coating film 6 is coated on the substrate 5 is not particularly restricted as long as a region in which all through-holes 2 are arranged can be covered. In the array 1, the whole surface of the substrate 5 may be coated. As shown in Fig. 1, for example, the hydrophobic coating film 6 may be formed so that all the through-holes 2 are covered and the substrate 5 is exposed in the periphery of the array 1.

A nucleic acid 3 which can form a prescribed receptor is loaded in contact with the substrate 5 in the through-holes 2 of the array 1. The nucleic acid 3 includes a nucleic acid including a gene which codes for the prescribed receptor. A plurality of nucleic acids 3 are loaded on the array 1 in contact with the substrate 5 and the nucleic acid 3 is loaded in each of the through-holes 2 of the array 1. Different types of nucleic acid 3 including a gene which codes for a prescribed receptor, for example, may be loaded in each of the through-holes 2.

The receptors indicate so-called transmembrane receptors, and include metabotropic receptors and ionotropic receptors. Examples of metabotropic receptors are G protein-coupled receptors, tyrosine kinase receptors, and guanylyl cyclase receptors. Examples of G protein-coupled receptors are olfactory receptors, muscarinic acetylcholine receptors, adenosine receptors, adrenaline receptors, GABA receptors (type B), angiotensin receptors, cannabinoid receptors, cholecystokinin receptors, dopamine receptors, glucagon receptors, histamine receptors, opioid receptors, secretin receptors, serotonin receptors, gastrin receptors, P2Y receptors and rhodopsin. Examples of tyrosine kinase receptors are insulin receptors, cell growth factor receptors and cytokine receptors. Examples of guanylyl cyclase receptors are GC-A, GC-B and GC-C.

Examples of ionotropic receptors are nicotinic acetylcholine receptors, glycine receptors, GABA receptors (type A, type C), glutamic acid receptors, serotonin receptor type 3, inositol trisphosphate (IP3) receptors, ryanodine receptors and P2X receptors.

It should be noted that the origin of a receptor coded by a gene included in the above nucleic acid is not particularly limited as long as the effects of the present invention are shown. Examples thereof can include animals, mammals, mice, primates or humans.

The nucleic acid 3 loaded in the through-holes 2 of the array 1 of the present invention is not particularly limited as long as the effects of the present invention are shown. Specific examples thereof can include DNA, RNA, PNA and the like. The nucleic acid 3 may further include lipid, polymer, virus or magnetic particles which are used to introduce the nucleic acid 3 into eukaryotic cells. The nucleic acid 3 may include, for example, a plasmid and a gene transfection reagent as described below. The nucleic acid 3 may also include a compound for promoting cell adhesion to increase adhesiveness between eukaryotic cells and the substrate 5.

In the case of a human-derived olfactory receptor, examples of the nucleic acid 3 loaded in the interior of the through-holes 2 of the array 1 of the present invention can include 404 genes specified by the NCBI accession numbers shown below: KP290534.1, NG_002151.2, NG_004125, NG_004272.4, NG_04652.2, NM_001001656, NM_001001657, NM_001001658, NM_001001659, NM_001001667, NM_001001674, NM_001001821, NM_001001824, NM_001001827.1, NM_001001912.2, NM_001001913, NM_001001914, NM_001001915, NM_001001916.2, NM_001001917.2, NM_001001918, NM_001001919, NM_001001920, NM_001001921, NM_001001922.2, NM_001001923, NM_001001952, NM_001001953.1, NM_001001954, NM_001001955.2, NM_001001956, NM_001001957, NM_001001957.2, NM_001001958.1, NM_001001959, NM_001001960.1, NM_001001961, NM_001001963.1, NM_001001964.1, NM_001001965, NM_001001966, NM_001001967, NM_001001968, NM_001002905.1, NM_001002907.1, NM_001002917.1, NM_001002918.1, NM_001002925, NM_001003443.2, NM_001003745.1, NM_001003750.1, NM_001004052.1, NM_001004058.2, NM_001004059.2, NM_001004063.2, NM_001004064.1, NM_001004124.2, NM_001004134.1, NM_001004135, NM_001004136.1, NM_001004137, NM_001004195.2, NM_001004297.2, NM_001004450, NM_001004451.1, NM_001004452.1, NM_001004453.2, NM_001004454.1, NM_001004456.1, NM_001004457, NM_001004458.1, NM_001004459.1, NM_001004460.1, NM_001004461, NM_001004461.1, NM_001004462, NM_001004462.1, NM_001004463.1, NM_001004464.1, NM_001004465, NM_001004466, NM_001004467.1, NM_001004469, NM_001004471.2, NM_001004472, NM_001004473, NM_001004474, NM_001004475, NM_001004476, NM_001004477, NM_001004478, NM_001004479, NM_001004480, NM_001004481, NM_001004482, NM_001004483, NM_001004484, NM_001004484, NM_001004485, NM_001004486, NM_001004487, NM_001004488, NM_001004489.2, NM_001004490, NM_001004491, NM_001004491.1, NM_001004492, NM_001004684, NM_001004685, NM_001004686, NM_001004686.2, NM_001004687, NM_001004688, NM_001004689, NM_001004690, NM_001004691, NM_001004692, NM_001004693, NM_001004694.2, NM_001004695, NM_001004696, NM_001004697, NM_001004698.2, NM_001004699.2, NM_001004700.2, NM_001004701.2, NM_001004702.1, NM_001004703.1, NM_001004704.1, NM_001004705, NM_001004706.1, NM_001004707.3, NM_001004708, NM_001004711, NM_001004712, NM_001004713.1, NM_001004714, NM_001004715, NM_001004717, NM_001004719, NM_001004723.2, NM_001004724, NM_001004725, NM_001004726, NM_001004727.1, NM_001004728, NM_001004729, NM_001004730.1, NM_001004731, NM_001004733.2, NM_001004734, NM_001004735, NM_001004736.3, NM_001004737, NM_001004738.1, NM_001004739, NM_001004740, NM_001004741.1, NM_001004742.2, NM_001004743, NM_001004744.1, NM_001004745.1, NM_001004746.1, NM_001004747.1, NM_001004748.1, NM_001004749.1, NM_001004750.1, NM_001004751, NM_001004752.1, NM_001004753, NM_001004754.0, NM_001004755.1, NM_001004756.2, NM_001004757.2, NM_001004758.1, NM_001004759.1, NM_001004760, NM_001005160, NM_001005161.3, NM_001005162.2, NM_001005163, NM_001005164.2, NM_001005165, NM_001005167, NM_001005168, NM_001005169, NM_001005170.2, NM_001005171.2, NM_001005172.2, NM_001005173.3, NM_001005174, NM_001005175.3, NM_001005177.3, NM_001005178, NM_001005179.2, NM_001005180.2, NM_001005181, NM_001005181.2, NM_001005182, NM_001005182, NM_001005183, NM_001005184, NM_001005185, NM_001005186.2, NM_001005187, NM_001005188, NM_001005189, NM_001005190.1, NM_001005191.2, NM_001005192.2, NM_001005193, NM_001005194.1, NM_001005195.1, NM_001005196.1, NM_001005197.1, NM_001005198.1, NM_001005199.1, NM_001005200.1, NM_001005201.1, NM_001005202.1, NM_001005203.2, NM_001005204, NM_001005205.2, NM_001005211, NM_001005212.3, NM_001005213, NM_001005216.3, NM_001005218.1, NM_001005222, NM_001005222.2, NM_001005224, NM_001005226.2, NM_001005234.1, NM_001005235, NM_001005236.3, NM_001005237.1, NM_001005238, NM_001005239, NM_001005240, NM_001005241.3, NM_001005243, NM_001005245, NM_001005270.4, NM_001005272.3, NM_001005274.1, NM_001005275.1, NM_001005276, NM_001005278, NM_001005279, NM_001005280.1, NM_001005281, NM_001005282, NM_001005283.2, NM_001005284, NM_001005285, NM_001005286, NM_001005287, NM_001005288, NM_001005288.2, NM_001005289, NM_001005323, NM_001005324, NM_001005325, NM_001005326, NM_001005327.2, NM_001005328, NM_001005329, NM_001005329.1, NM_001005334, NM_001005338, NM_001005465.1, NM_001005466.2, NM_001005467.1, NM_001005468.1, NM_001005469, NM_001005470.1, NM_001005471, NM_001005479.1, NM_001005480.2, NM_001005482, NM_001005483, NM_001005484, NM_001005484, NM_001005486, NM_001005487.1, NM_001005489, NM_001005489, NM_001005490, NM_001005490.1, NM_001005491.1, NM_001005492, NM_001005493, NM_001005494, NM_001005495, NM_001005496, NM_001005497, NM_001005499, NM_001005500, NM_001005501, NM_001005503, NM_001005504, NM_001005504, NM_001005512.2, NM_001005513.1, NM_001005514, NM_001005515, NM_001005516, NM_001005517, NM_001005518, NM_001005519.2, NM_001005522, NM_001005566.2, NM_001005567, NM_001005853, NM_001013355.1, NM_001013356.2, NM_001013357.1, NM_001013358.2, NM_001079935, NM_001146033.1, NM_001160325, NM_001197287, NM_001258283, NM_001258284, NM_001258285, NM_001291438.1, NM_001317107.1, NM_001348224.1, NM_001348266.1, NM_001348271.1, NM_001348273.1, NM_001348286.1, NM_002548.2, NM_002550.2, NM_002551.3, NM_003552.3, NM_003553.2, NM_003554.2, NM_003555, NM_003696.2, NM_003697, NM_003700, NM_006637, NM_007160.3, NM_012351.2, NM_012352.2, NM_012353., NM_012360, NM_012363.1, NM_012363.1., NM_012364.1, NM_012365, NM_012367, NM_012368.2, NM_012369.2, NM_012373.2, NM_012374, NM_012375, NM_012377, NM_012378.1, NM_013936.3, NM_013937.3, NM_013938, NM_013939.2, NM_013940.2, NM_013941., NM_014565.2, NM_014566, NM_01750, NM_017506.1, NM_019897.2, NM_030774.3, NM_030876.5, NM_030883.4, NM_030901.1, NM_030903.3, NM_030904, NM_030905.2, NM_030908.2, NM_030946, NM_030959.2, NM_033057.2, NM_033179.2, NM_033180.4, NM_054104, NM_054105, NM_054106, NM_054107.1, NM_080859.1, NM_152430.3, NM_153444, NM_153445, NM_172194, NM_173351, NM_175883.2, NM_175911.3, NM_178168.1, NM_198074.4, NM_198944.1, NM_205859, NM_206880, NM_206899, NM_207186.2 or NM_207374.3.

An aspect of the nucleic acid 3 loaded on the array 1 of the present invention is not particularly limited as long as the effects of the present invention are shown.

Specifically, a method for arranging the above nucleic acid 3 on the substrate 5 in the through-holes 2 is not particularly limited as long as the effects of the present invention are shown. Examples thereof can include a method for printing the nucleic acid 3 in the form of dot. A known apparatus or an equivalent thereof provided with a piezoelectric element (including MEMS), in which a solution is not easily degenerated in printing, such as is provided in the examples below, e.g. an inkjet printer, can be used in printing as described above. The nucleic acid 3 in the through-holes 2 is provided in a size of typically 10 mm or more and 300 mm or less in diameter.

When the array 1 of the present invention is brought into contact with cells, a gene which codes for a receptor can be introduced into the cells. Therefore, the receptor can be expressed in the cells. As an example, an olfactory receptor derived from an animal, a mammal or a human can be expressed.

It should be noted that the basic constitution of the array 1 of the present invention has been described; however, various applications can be made. For example, a region to write identification numbers may be provided in the array 1 using a region in which the substrate 5 is not covered by the hydrophobic coating film 6, or a region of the hydrophobic coating film 6 other than the through-holes 2. Specifically, alphanumerics determined in advance such as a one dimensional barcode or a two dimensional barcode (including QR code (registered trademark)) may be written in a form which can be optically, magnetically or electronically read. The codes may be different unique codes in each of the arrays 1, or may be the same fixed codes in each of the arrays 1. The identification number can also include arrangement information of the nucleic acid 3 in the array 1. For example, information about the X-coordinate and Y-coordinate of the nucleic acid 3 in the array 1, and the constitution (sequence) of the nucleic acid placed thereon may be coded in the form of matrix and recorded. The region in which to write the identification number may also be a blank region which can be drawn on by handwriting or laser processing (e.g. a roughened region, or a region to which a material colored by laser light is applied).

In addition, the array 1 may be held in a film bag of a synthetic resin such as polyethylene or polypropylene, or a container of polystyrene, glass or the like for stable storage during waiting time until eukaryotic cells 4 are loaded. The interior of the bag or container to hold it may also be vacuous, decompressed or filled with an inert gas such as nitrogen or argon.

Next, the form and arrangement of the through-holes 2 will be illustrated using Fig. 2. Fig. 2 is a macroscopic plan view which shows the arrangement of the through-holes 2 of the array 1. In the description, the above-described substrate 5, hydrophobic coating film 6 and nucleic acid 3 are not shown. Specifically, Fig. 2 is a plan view when the array 1 is in the form of cuboid, and the longitudinal direction of the array 1 is the X-axis and the orthogonal direction (the direction perpendicular to the X-axis) is the Y-axis. A plurality of through-holes 2 are provided in the array 1. The form is optional and a polygon such as a triangle or a square or a circle can be used. The circle may be also an ellipse. The form of the through-holes 2 is most desirably a circle, particularly a perfect circle. It should be noted that Fig. 2 shows a case where the form of the through-holes 2 is a perfect circle and 25 through-holes are arranged in the array 1.

As the size of the through-holes 2, the average value of the cross-sectional area thereof is desirably 0.125 mm² or more per through-hole. The average value of the cross-sectional area is obtained by calculating all the areas of the through-holes 2 in the height direction (depth direction) of the through-holes 2, a direction perpendicular to the central axis thereof, and averaging the obtained values. That is, when the form of the through-holes 2 is a perfect circle in an overhead view, the diameter thereof (corresponding to the diameter D of the through-hole 2B in Fig. 2) is, but not limited to, 0.4 mm or more. When the average value of the cross-sectional area is 0.125 mm² or more as the size of the through-holes 2, 400 or more eukaryotic cells can be brought into contact with one of the through-holes 2, and the number of cells suitable for measurement is easily secured even when transformation of eukaryotic cells described below varies depending on individuals. For example, in the case of the through-holes 2 with an average cross-sectional area value of less than 0.125 mm², because the number of cells in the through-holes 2 is small, there exist difficulties in that the sufficient number of transformed cells cannot be secured or the sufficient number of cells for statistical processing of the activation degree of a receptor cannot be secured. It should be noted that in a case where the prescribed receptor is an olfactory receptor, 10 or more of 400 or more eukaryotic cells, which correspond to about 2.5%, are expressed in one of the through-holes 2, and the number of cells required for statistical processing of the activation degree of the receptor is obtained.

It is desired that the nucleic acids 3 which code for a prescribed receptor be all arranged in one array 1. That is, when receptors corresponding to each nucleic acid 3 are expressed by a plurality of nucleic acids 3, it is desired that the nucleic acids 3 be arranged one by one in the through-holes 2 of the array 1. In other words, that the nucleic acids 3 including genes which code for prescribed different receptors be provided in each through-hole 2. As an example, when a receptor is a human olfactory receptor, there exist about 400 types of receptor. At this time, because an odor perception ability varies depending on environmental elements such as temperature, air volume and atmospheric distribution (concentration distribution of molecules in the atmosphere caused by air current), about 400 nucleic acids 3 which are different from each other and which correspond to a set of receptors, are arranged in one array, and most desirably all the receptors in the set are comprehensively measured by one measurement. At this time, the array 1 has about 400 through-holes 2.

In order to measure an activation degree in real time, particularly continuously in seconds, in a measurement method described below, it is necessary to simultaneously measure several hundred (e.g. 200 or more) through-holes 2. In particular, for measurements using fluorescence, the size of the through-holes 2 and the central clearance (pitch) between the through-holes 2 is restricted because the observation field of the objective lens of the optical system (e.g. an optical microscope) used for fluorescence measurement is limited. In order to simultaneously measure, for example, 300 or more, particularly about 400 through-holes 2, it is desirable that the average value of the cross-sectional area of the through-holes 2 be typically 0.283 mm² or less. When the form of the through-holes 2 is a perfect circle, the diameter thereof (corresponding to the diameter D of the through-hole 2B in Fig. 2) is 0.6 mm or less. It is also desirable that the central clearance between each spot (pitch P1 on the X axis obtained by the through-holes 2B and 2A, or pitch P2 on the Y axis obtained by the through-holes 2B and 2C in Fig. 2) be 0.6 mm or more and 0.8 mm or less. It is also desirable that P1 = P2. It is most desirable that P1 = P2 = 0.6 mm or more and 0.8 mm or less.

That is, in a case where a fluorescence value is used for measurement, for example when the form of the through-holes 2 is a perfect circle, the diameter D is desirably 0.4 mm or more and 0.6 mm or less, and the average pitch P1 and the average pitch P2 are desirably 0.6 mm or more and 0.8 mm or less. Here, the diameter D may be 0.42 mm or more, 0.44 mm or more, 0.46 mm or more, 0.48 mm or more, or 0.50 mm or more, and may be 0.58 mm or less, 0.56 mm or less, 0.54 mm or less, or 0.52 mm or less. In addition, the average pitches P1 and P2 may be each independently 0.64 mm or more, 0.68 mm or more, 0.72 mm or more, or 0.76 mm or more, and may be 0.76 mm or less, 0.72 mm or less, or 0.68 mm or less. In one embodiment, the form of the through-holes 2 is a perfect circle, the diameter D thereof is 0.5 mm, and the average pitch is P1 = P2 = 0.7 mm. Using these numerical values, 22 through-holes 2 on the X axis, 18 on the Y axis and a total of 396 through-holes 2 will be arranged so that the centers of the through-holes will be close together in a range of 15.58 mm x 12.82 mm in the array 1.

### Array holding eukaryotic cells

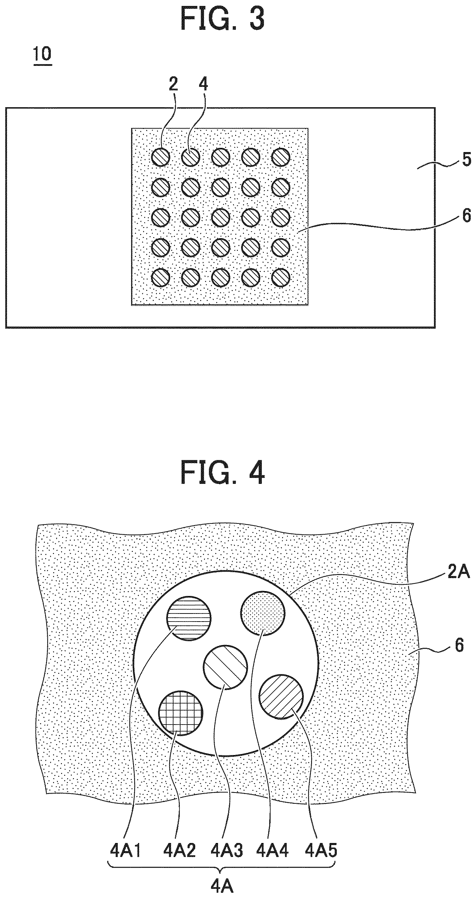

An array holding eukaryotic cells of the present invention (hereinafter can be referred to as eukaryotic cell array 10) will be illustrated using Fig. 3, Fig. 2 and Fig. 4. As shown in Fig. 3, the array 10 holding eukaryotic cells of the present invention is based on the array 1, and includes a substrate 5, a hydrophobic coating film 6 on the substrate 5 and a nucleic acid 3. A plurality of through-holes 2 are provided in the hydrophobic coating film 6 and the form and arrangement of the through-holes 2 are the same as of the array 1 (shown in Fig. 2). Eukaryotic cell regions 4 are provided in the through-holes 2. Fig. 4 is a schematic plan view in which one of the through-holes 2 is enlarged, and the through-hole 2A and eukaryotic cell regions 4A are provided in the hydrophobic coating film 6. A plurality of eukaryotic cells are provided so as to overlap the nucleic acid 3 loaded on the substrate 5 in the eukaryotic cell region 4A (5 eukaryotic cells 4A1 to 4A5 are schematically shown and the nucleic acid 3 is not shown in Fig. 4). In Fig. 4, a case of 5 eukaryotic cells in one through-hole 2 is shown; however, the number of eukaryotic cells may be 2 or more, 5 or more, 10 or more, 50 or more, 100 or more, 200 or more, 300 or more, 400 or more, 500 or more, 600 or more, 700 or more, 800 or more, 1000 or more, or 2000 or more. The number of eukaryotic cells may be also 10000 or less, 9000 or less, 8000 or less, 7000 or less, 6000 or less, 5000 or less, 4000 or less, 3000 or less, 2000 or less, or 1000 or less. The eukaryotic cells may be eukaryotic cells derived from, for example, animals, mammals, primates or humans. In one embodiment the eukaryotic cells are eukaryotic cells derived from humans and the number thereof is 400 or more and 1000 or less.

As described above, a plurality of eukaryotic cells are loaded on one nucleic acid 3 in a through-hole 2, and therefore it is possible to reduce the risk that a specific nucleic acid 3 cannot be measured even when a receptor is expressed or not expressed in eukaryotic cells based on the nucleic acid 3. In one embodiment, for example, when the nucleic acid 3 is an olfactory receptor, the eukaryotic cells are eukaryotic cells derived from humans, and 400 eukaryotic cells are arranged in one through-hole 2 (one nucleic acid 3), about 40 eukaryotic cells, which correspond to about 10%, are expressed in a state in which a measurement can be made. Then, 40 eukaryotic cells are each individually recognized and handled as information, and thus a measurement system by which the activation degree of about 400 types of human olfactory receptor to a test substance are all measured can be established.

Incidentally, when a receptor is used as a device, there are two methods for expressing the receptor in cells: stable expression (stable transfection), in which cell genes are altered, and transient expression (transient transfection), which is carried out by introducing a plasmid into cells. In the case of stable expression, because information of intracellular genes is altered, a receptor can be permanently used; however, because the yield of gene expression is bad, a prescribed receptor is selected from those after cell culture and loaded onto a device. Therefore, enormous efforts and costs are required to load stably expressed receptors, for example several hundreds of receptors, onto one device. On the other hand, in the case of transient expression, expressed cells can be maintained and used only in a short period of time, typically about one week; however, the cells can be relatively easily made, and can be loaded onto one device. While cells are relatively easily made, the degree of expression varies.

The eukaryotic cell array 10 of the present invention has excellent compatibility with a means for making a receptor by such transient expression. That is, 400 eukaryotic cells can be arranged with respect to the nucleic acid 3 in the through-hole 2, for example, (e.g. 400 eukaryotic cells are held in the through-hole 2A), and thus about 10 eukaryotic cells express receptors which can be measured even if expression by transient expression varies. The approximately 10 receptors have different efficiencies in the measurement of activation degrees; however, as long as 10 eukaryotic cells are individually recognized one by one in a measurement system and the activation degree of each cell normalized by a calculation method (A) or (B) for activation degrees described below is measured and calculated, information about the activation degree of each receptor can be appropriately provided.

A method for getting eukaryotic cells expressing a receptor derived from a mammal is not particularly limited as long as the effects of the present invention are shown. Primary cultured cells including olfactory cells derived from a mouse, for example, can be used. Cells of the present invention mentioned below can be also used.

It should be noted that a means for confirming the above expression level is not particularly limited as long as the effects of the present invention are shown. Examples thereof can include a means based on known techniques such as western blot.

The type of the above receptor expressed in eukaryotic cells is not particularly limited as long as the effects of the present invention are shown. For example, a type of olfactory receptor can be expressed, or a plurality of types of olfactory receptor can be also expressed in one cell. It is preferred that a type of olfactory receptor be expressed in one cell.

In the case of 4 types of identified olfactory receptor derived from a mammal: a, b, g and d, examples of preferred aspects can include an aspect in which a and b are expressed in eukaryotic cells A, b and g are expressed in eukaryotic cells B, and d is expressed in eukaryotic cells C, and the total amounts of a, b, g and d each expressed in the eukaryotic cells A, B and C are the same, and eukaryotic cells A, B and C are expressed in the same system.

A receptor expressed in eukaryotic cells can be identified before the calculation method (A) or calculation method (B) of the present invention described below, or can be identified after the method. In the case of identification before the method is implemented, a gene allowing an already identified olfactory receptor derived from a mammal to be expressed may be introduced into the above eukaryotic cells, and in the case of identification after implementation of the method, an olfactory receptor included in cells can be easily identified by combining known techniques for gene analysis with the cells after the activation degrees described hereafter are measured by the calculation method (A) or calculation method (B).

As for the eukaryotic cell array 10 of the present invention, the basic constitution of the eukaryotic cell array 10 has been illustrated. It should be noted, however, that various applications can be made. For example, a region in which to write identification numbers can be provided in the eukaryotic cell array 10 by using a region in which the substrate 5 is not covered by the hydrophobic coating film 6, or a region of the hydrophobic coating film 6 other than the through-holes 2. Specifically, alphanumerics determined in advance such as a one dimensional barcode or a two dimensional barcode (including QR code (registered trademark)) may be written in a form which can be optically, magnetically or electronically read. The codes may be different unique codes in each of the eukaryotic cell arrays 10, or may be fixed codes in each of the eukaryotic cell arrays 10. In addition, the identification number may include arrangement information of the nucleic acid 3 in the array 1. For example, information about the X-coordinate and Y-coordinate of the nucleic acid 3 in the array 1, and the constitution (sequence) of the nucleic acid placed thereon may be coded in the form of a matrix and recorded. The region in which to write the identification number may also be a blank region which can be drawn on by handwriting or laser processing (e.g. a roughened region, or a region to which a material colored by laser light is applied). In addition, the eukaryotic cell array 10 may be held in a film bag of a synthetic resin such as polyethylene or polypropylene, or a container of polystyrene, glass or the like for stable storage. The interior of the bag or container to hold it may be vacuous, decompressed or filled with an inert gas such as nitrogen or argon.

### Eukaryotic cell

The eukaryotic cells of the present invention (e.g. 4A1 to 4A5 in Fig. 4) are eukaryotic cells expressing CNGA2 and GNAL as foreign genes when the prescribed receptor is an olfactory receptor. The eukaryotic cells are not particularly limited as long as the effects of the present invention are shown. Examples thereof can include yeast cells, animal cells, insect cells, mammalian cells, primate cells and the like. Among the above eukaryotic cells, mammalian cells are preferred, and line cells typified by immortalized HEK 293 cells, CHO cells or HeLa cells or the like, which are particularly easily handled, are preferred. In addition, Rolf Ba. T cells (Glia 16:247 (1996)) derived from rat olfactory sensory neurons can be also used.

CNGA2 is a gene which codes for a protein, called Cyclic Nucleotide Gated Channel Alpha 2, existing in cell membranes and this depends on cAMP to function as a calcium channel.

The origin of the above CNGA2 is not particularly limited as long as the effects of the present invention are shown. Examples thereof are mammals. Among these, examples thereof can include mice and humans.

The amino acid sequence of the protein coded by CNGA2 is not particularly limited as long as the effects of the present invention are shown. Examples thereof can include an amino acid sequence coded by a base sequence shown in SEQ ID NO:1.

In addition, mutants of the amino acid sequence coded by the base sequence shown in SEQ ID NO:1 can be also included in the above CNGA2 as long as the mutants can function as a calcium ion channel. The amino acid homology between before and after mutation is, for example, 80% or more, preferably 85% or more, preferably 90% or more, preferably 91% or more, and further preferably 95% or more. Such mutation includes aspects such as substitution, deletion and insertion. Therefore, splice mutants can be also included in the above mutants.

Among mutants of the protein coded by the above CNGA2, preferred is a mutant in which the 342^{nd} glutamic acid of the amino acid sequence of human-derived CNGA2 coded by the base sequence shown in SEQ ID NO:1 is changed to glycine, the 460^{th} cysteine to tryptophan, and the 583^{rd} glutamic acid to methionine, from the viewpoint of more efficiently functioning as a calcium ion channel.

GNAL expressed in cells of the present invention is a gene which codes for a protein called heterotrimeric Guanine nucleotide-binding protein (G protein) alpha subunit L, and this is a G protein responsible for signal transduction caused by stimulation to an olfactory receptor in cells expressing the olfactory receptor of olfactory sensory neurons.

The origin of the above GNAL is not particularly limited as long as the effects of the present invention are shown. Examples thereof can include mammals. Among these, examples thereof can include mice and humans.

The amino acid sequence of GNAL is not particularly limited as long as the effects of the present invention are shown. Examples thereof can include an amino acid sequence coded by a base sequence shown in SEQ ID NO:2. In addition, mutants of the amino acid sequence coded by the base sequence shown in SEQ ID NO:2 can be also included in the above GNAL as long as the mutants can be responsible for signal transduction caused by stimulation to an olfactory receptor in cells expressing the olfactory receptor of olfactory sensory neurons. The amino acid homology between before and after mutation is, for example, 80% or more, preferably 85% or more, preferably 90% or more, preferably 91% or more, and further preferably 95% or more. Such mutation includes aspects such as substitution, deletion and insertion. Therefore, splice mutants can be also included in the above mutants.

The cells of the present invention have adenylate cyclase and the beta subunit and gamma subunit of heterotrimeric G proteins in addition to proteins coded by the above genes.

It is further preferred that the above cells of the present invention be cells expressing CNGA4 and/or CNGBlb as foreign genes.

CNGA4 is a gene which codes for a protein, called Cyclic Nucleotide Gated Channel Alpha 4, existing in cell membranes and this also functions as a calcium ion channel as in the case of the protein coded by the above CNGA2.

The origin of the above CNGA4 is not particularly limited as long as the effects of the present invention are shown. Examples thereof can include mammals. Among these, examples thereof can include mice and humans.

The amino acid sequence of the protein coded by CNGA4 is not particularly limited as long as the effects of the present invention are shown. Examples thereof can include an amino acid sequence coded by a base sequence shown in SEQ ID NO:3.

In addition, mutants of the amino acid sequence coded by the base sequence shown in SEQ ID NO:3 can be also included in the above CNGA4 as long as the mutants can function as a calcium ion channel. The amino acid homology before and after mutation is, for example, 80% or more, preferably 85% or more, preferably 90% or more, preferably 91% or more, and further preferably 95% or more. Such mutation includes aspects such as substitution, deletion and insertion. Therefore, splice mutants can also be included in the above mutants.

CNGBlb is a gene which codes for a protein, called Cyclic Nucleotide Gated Channel Beta 1b, existing in cell membranes and this also functions as a calcium ion channel as in the case of the proteins coded by the above CNGA2 and CNGA4.

The origin of the above CNGBlb is not particularly limited as long as the effects of the present invention are shown. Examples thereof can include mammals. Among these, examples thereof can include mice and humans.

The amino acid sequence of the protein coded by CNGBlb is not particularly limited as long as the effects of the present invention are shown. Examples thereof can include an amino acid sequence coded by a base sequence shown in SEQ ID NO:4.

In addition, mutants of the amino acid sequence coded by the base sequence shown in SEQ ID NO:4 can be also included in the above CNGBlb as long as the mutants can function as a calcium ion channel. The amino acid homology before and after mutation is, for example, 80% or more, preferably 85% or more, preferably 90% or more, preferably 91% or more, and further preferably 95% or more. Such mutation includes aspects such as substitution, deletion and insertion. Therefore, splice mutants can also be included in the above mutants.

When CNGA2, CNGA4 and CNGBlb are expressed in cells of the present invention, it is preferred that these three be expressed at a ratio of 2 : 1 : 1 respectively because sensitivity to cAMP increased in signal transduction in cells caused by stimulation to an olfactory receptor is increased.

It is preferred that the above cells of the present invention be cells expressing a gene which codes for a protein which can transfer an olfactory receptor expressed in the cells from the endoplasmic reticulum membrane to the cell membrane to raise the efficiency of presentation of the olfactory receptor to the surface of the cells.

The above protein which can transfer an olfactory receptor expressed in cells from the endoplasmic reticulum membrane to the cell membrane to raise the efficiency of presentation of the olfactory receptor to the surface of the cells is not particularly limited as long as the effects of the present invention are shown. Examples thereof can include proteins such as RTP1, RTP2 and REEP1.

The amino acid sequence of RTP1 is not particularly limited as long as the effects of the present invention are shown. Examples thereof can include an amino acid sequence coded by a base sequence shown in SEQ ID NO:5.

In addition, mutants of the amino acid sequence coded by the base sequence shown in SEQ ID NO:5 can be also included in the above RTP1 as long as the mutants can transfer an olfactory receptor expressed in cells from the endoplasmic reticulum membrane to the cell membrane to raise the efficiency of presentation of the olfactory receptor to the surface of the cells. The amino acid homology before and after mutation is, for example, 80% or more, preferably 85% or more, preferably 90% or more, preferably 91% or more, and further preferably 95% or more. Such mutation includes aspects such as substitution, deletion and insertion. Therefore, splice mutants can be also included in the above mutants.

The splice mutants of the above RTP1 are not particularly limited as long as the effects of the present invention are shown. Examples thereof can include RTP1L, RTP1S and the like. Among these, examples of RTP1S can include an amino acid sequence coded by a base sequence shown in SEQ ID NO:6. The amino acid sequence of RTP2 is not particularly limited as long as the effects of the present invention are shown. Examples thereof can include an amino acid sequence coded by a base sequence shown in SEQ ID NO:7.

In addition, mutants of the amino acid sequence coded by the base sequence shown in SEQ ID NO:7 can be also included in the above RTP2 as long as the mutants can transfer an olfactory receptor expressed in cells from the endoplasmic reticulum membrane to the cell membrane to raise the efficiency of presentation of the olfactory receptor to the surface of the cells. The amino acid homology before and after mutation is, for example, 80% or more, preferably 85% or more, preferably 90% or more, preferably 91% or more, and further preferably 95% or more. Such mutation includes aspects such as substitution, deletion and insertion. Therefore, splice mutants can be also included in the above mutants.

The amino acid sequence of REEP1 is not particularly limited as long as the effects of the present invention are shown. Examples thereof can include an amino acid sequence coded by a base sequence shown in SEQ ID NO:8.

In addition, mutants of the amino acid sequence coded by the base sequence shown in SEQ ID NO:8 can be also included in the above REEP1 as long as the mutants can transfer an olfactory receptor expressed in cells from the endoplasmic reticulum membrane to the cell membrane to raise the efficiency of presentation of the olfactory receptor to the surface of the cells. The amino acid homology before and after mutation is, for example, 80% or more, preferably 85% or more, preferably 90% or more, preferably 91% or more, and further preferably 95% or more. Such mutation includes aspects such as substitution, deletion and insertion. Therefore, splice mutants can also be included in the above mutants.

In the upstream of the above genes which code for proteins which can transfer an olfactory receptor expressed in cells from the endoplasmic reticulum membrane to the cell membrane to raise the efficiency of presentation of the olfactory receptor to the surface of the cells, a promotor which does not have an epigenetic effect on these genes is preferably arranged.

Such a promoter is not particularly limited as long as the effects of the present invention are shown. Examples thereof can include Elongation Factor-la (EF-1a).

The origin of the promoter which does not have an epigenetic effect is not particularly limited as long as the effects of the present invention are shown. Examples thereof can include mice or humans.

The eukaryotic cells of the present invention can be produced by introducing a vector having CNGA2 and GNAL (this can be referred to as "Vector A" in the description) into eukaryotic cells. In addition, into cells produced as described above, a vector having CNGA4 and/or CNGBlb (this can be referred to as "Vector B" in the description) can be introduced. Then, a vector having a gene which codes for a protein which can transfer an olfactory receptor expressed in cells from the endoplasmic reticulum membrane to the cell membrane to raise the efficiency of presentation of the olfactory receptor to the surface of the cells (this can be referred to as "Vector C" in the description) can be introduced.

The vector of the present invention is any of the above vectors A, B and C. Eukaryotic cells, each having a gene which can be expressed by introducing these vectors, are not particularly limited. Examples thereof can include yeast cells, mammalian cells and insect cells. Among these, mammalian cells are preferred. The vectors constructed above can produce a recombinant virus using a known virus, and such recombinant virus can induce transient expression in eukaryotic cells. Examples of known viruses can include adenoviruses, retroviruses, adeno-associated viruses, and baculoviruses.

It should be noted that the above vectors A, B and C can also induce stable expression by adjusting conditions. Stable expression is achieved, for example, by providing not less than a certain number of regions in which a gene coding for an olfactory receptor is arranged, onto the chromosome of cells of the present invention. The above region is a region which reacts with a recombinase provided in a prescribed site on the chromosome.

Such recombinase is not particularly limited as long as the effects of the present invention are shown. Examples thereof can include Flp, Cre and the like. It should be noted that examples of regions which react with these recombinases can include Frt and LoxP respectively.

As mentioned above, not less than a certain number of regions into which a gene coding for an olfactory receptor is incorporated exist in the chromosome of cells of the present invention, and therefore a gene coding for an olfactory receptor can be incorporated only into the regions. For example, the number of genes coding for an olfactory receptor incorporated can be adjusted depending on the number of the regions, and thus the amount of olfactory receptors expressed in the cells can be also adjusted.

By way of the above cells of the present invention, signal transduction caused by stimulation to an olfactory receptor in olfactory sensory neurons can be achieved. Therefore, by using the cells of the present invention, calculation methods (A) and (B) for activation degrees of the present invention described below can be suitably carried out.

In order for these receptors to be expressed on cell membranes with the correct topology, the above olfactory receptor is preferably fused to the amino acids (MNGTEGPNFYVPFSNKTGVV) shown in SEQ ID NO:9, which are 20 amino acid residues in the N terminus of a Rhodopsin molecule, and these are incorporated into the cells of the present invention and are expressed together.

### Calculation method for degree of activation

As methods for calculating the degree of activation of a receptor by a test substance (this can be referred to as ligand in the description) of the present invention (hereinafter this can be referred to as "calculation method" in the description), there are two calculation methods: calculation method (A) and calculation method (B).

### Calculation method (A) for degree of activation

The calculation method (A) for the degree of activation is a method including the following steps 1 to 3.

### (Step 1)

Step of measuring, when a test substance is brought into contact with eukaryotic cells expressing a receptor, the amount of ion taken into the cells.

### (Step 2)

Step of measuring, when the same cells as used for measurement in the step 1 are depolarized, the amount of ion in the cells.

### (Step 3)

Step of calculating the ratio of a numerical value measured in step 1 and a numerical value measured in step 2.

Step 1 and step 2 may be carried out before step 3, and the order of step 1 and step 2 is not particularly limited as long as the order does not affect the effects obtained by the calculation method of the present invention. In view of the degree of reliability of numerical values obtained by the calculation method of the present invention, step 2 is preferably carried out after step 1.

The above test substance is not particularly limited as long as the effects of the present invention are shown. A substance including an odor component which is not generally recognized by animals such as humans, for example, can be also included in the test substance in the calculation method of the present invention.

The category of the above test substance is not particularly limited as long as the effects of the present invention are shown. The test substance can be, for example, a pure substance or a mixture. In addition, the pure substance is not particularly limited as to whether it is a single body or a compound.

The form of the above test substance is not particularly limited as long as the effects of the present invention are shown. The test substance may be in the form of, for example, a gas, a solid or a liquid.

The above receptor is not particularly limited as long as the effects of the present invention are shown. The receptor may be, for example, a prescribed receptor to which the nucleic acid 3 in the above-described array 1 belongs, which indicates a so-called transmembrane receptor, and can include, for example, metabotropic receptors, and ionotropic receptors.

Examples of metabotropic receptors are G protein-coupled receptors, tyrosine kinase receptors, and guanylyl cyclase receptors. Examples of G protein-coupled receptors are olfactory receptors, muscarinic acetylcholine receptors, adenosine receptors, adrenaline receptors, GABA receptors (type B), angiotensin receptors, cannabinoid receptors, cholecystokinin receptors, dopamine receptors, glucagon receptors, histamine receptors, opioid receptors, secretin receptors, serotonin receptors, gastrin receptors, P2Y receptors and rhodopsin. Examples of tyrosine kinase receptors are insulin receptors, cell growth factor receptors and cytokine receptors. Examples of guanylyl cyclase receptors are GC-A, GC-B and GC-C.

Examples of ionotropic receptors are nicotinic acetylcholine receptors, glycine receptors, GABA receptors (type A, type C), glutamic acid receptors, serotonin receptor type 3, inositol trisphosphate (IP3) receptors, ryanodine receptors and P2X receptors.

The types of target ion in the step of measuring the amount of ion in cells in the above step 1 and step 2 are different depending on the types of the above receptor. When the receptor is an olfactory receptor, examples thereof can include calcium ion, and when the receptor is a nicotinic acetylcholine receptor, examples thereof can include sodium ion, and when the receptor is a glycine receptor, examples thereof can include chloride ion.

When the receptor is an olfactory receptor, a contact method with a test substance will now be illustrated. When the test substance is in the form of a solid, by applying a solution obtained using a known solvent which can elute an odor component included in the substance to eukaryotic cells, the test substance can be brought into contact with eukaryotic cells. In addition, when the test substance is in the form of a gas, by applying a solution obtained using a known solvent which can absorb an odor component included in the test substance to eukaryotic cells, the test substance can be brought into contact with eukaryotic cells.

The above known solvent is not particularly limited as long as the solvent does not respond to an olfactory receptor derived from a mammal by the method of the present invention. Examples thereof can include water, a buffer solution, DMSO, methanol, ethanol, a medium, Ringer solution and the like. Each step in the calculation method of the present invention will be described in detail.

### Step 1

Step 1 in the calculation method of the present invention is a step of measuring, when a test substance is brought into contact with eukaryotic cells expressing a receptor, the amount of ion taken into the cells. A case where the receptor is an olfactory receptor derived from a mammal and ion taken into the cells is calcium ion will be illustrated below.

In step 1, the medium, culture conditions and the like to maintain eukaryotic cells expressing an olfactory receptor derived from a mammal are not particularly limited as long as they do not have an influence on the measurement of the amount of calcium ion taken in the eukaryotic cells in steps 1 and 2. Examples thereof can include commonly media, temperatures and carbon dioxide concentrations in which cells derived from mammals can be cultured.

It should be noted that a site where the amount of calcium ion taken into eukaryotic cells expressing an olfactory receptor derived from a mammal is measured in step 1 is not particularly limited as long as the above medium and culture environment can be maintained. Examples thereof can include sites suitable for cell cultures such as on a dish, a plate, a multiwell plate, a chamber and an array. From the viewpoint of carrying out the calculation method of the present invention with high throughput, measurement is preferably carried out on a multiwell, a chamber provided with a multiwell and an array. At this time, it is preferred that olfactory receptors derived from a mammal, which are expressed by cells stored in each well, be different from each other.

The means for measuring the amount of calcium ions taken in eukaryotic cells expressing an olfactory receptor derived from a mammal in step 1 is not particularly limited as long as the effects of the present invention are shown. Examples thereof can include a means using a calcium ion-measuring pigment, a means using a calcium ion-binding protein, and the like.

The pigment used in the above means is not particularly limited as long as the effects of the present invention are shown. Examples thereof can include fluorescent pigments typified by Fluo-4, Rhod-3, Fluo-3, Fura-2, Indo-1, Quin-2, Rhod-2, or Fluo-8 or the like. These fluorescent pigments are preferably AM derivatives (protected by the acetoxymethyl group) which increase cell membrane permeability.

It should be noted that because AM derivatives can be in the form of granules in water, for the purpose of allowing cells to take these in, a surface activating agent such as Pluronic F-127 or Cremophor EL may be used. Among the above pigments, Fluo-3, Fluo-4, Quin-2 or the like is preferably used from the viewpoint of being able to measure the amount of calcium ion taken into cells over time, being able to efficiently carry out measurement, having characteristics of excitation at a single wavelength and fluorescence at a single wavelength, which are highly versatile around a microscope B excitation, and having a calcium ion complex dissociation constant (Kd) of 1 nmol/ml or less. A specific method for measuring the amount of calcium ion taken into cells using these pigments is not particularly limited. Specifically, examples thereof can include a method for measuring fluorescence values emitted by a pigment depending on the binding state of the pigment and calcium ion after being brought into contact.

The calcium ion-binding protein used in the above means is not particularly limited as long as the effects of the present invention are shown. Examples thereof can include fluorescent proteins which are bound to calcium ion typified by Geco such as G-Geco, B-Geco, R-Geco, GEX-Geco or GEM-Geco; Case 12, CEPIA, Aequorin, Cameleon, Pericam or GcaMP or the like. A specific method for measuring the amount of calcium ion taken into cells using these calcium ion-binding proteins is not particularly limited. Specifically, examples thereof can include a method for measuring fluorescence values emitted by a calcium ion-binding protein depending on the binding state of the calcium ion-binding protein and calcium ion after being brought into contact.

The method for measuring the amount of calcium ions in step 1 is not particularly limited as long as the effects of the present invention are shown. In a case where the means for measuring the amount of calcium ions is a means using a pigment, for example, a numerical value obtained after a test substance is brought into contact with cells may be directly measured as the amount of calcium ions based on the numerical value (fluorescence value) when cells are not stimulated.

Specifically, a means for finding the above numerical value is not particularly limited as long as the effects of the present invention are shown. For example, when the amount of calcium ions is measured using the above pigment, the difference in a one dimensional numerical value between a fluorescence value measured when cells are not stimulated (this can be referred to as "background value" in this description) and a fluorescence value measured after stimulation may be a numerical value found in step 1.

In addition, the numerical value in step 1 can be also found based on the difference in a two dimensional numerical value between a fluorescence value measured when cells are not stimulated (background value) and a fluorescence value measured after stimulation.

### Step 2

Step 1 in the calculation method of the present invention is a step of measuring the amount of calcium ions when the same cells as used for measurement in step 1 are depolarized.

The means for depolarizing the same cells as used for measurement in step 1 in step 2 is not particularly limited as long as the effects of the present invention are shown. Examples thereof can include a means for bringing a potassium compound or ionophore into contact with the above cells.

The above potassium compound is not particularly limited as long as the effects of the present invention are shown. Examples thereof can include inorganic potassium compounds typified by potassium chloride, potassium bromide, potassium hydroxide, potassium amide, potassium fluoride, potassium nitrate, potassium nitrite, potassium sulfate, potassium sulfite, potassium carbonate, potassium hydrogen carbonate, monopotassium phosphate, dipotassium phosphate, tripotassium phosphate, potassium chromate, potassium dichromate, potassium pyrophosphate, potassium metaphosphate, potassium chlorate, potassium perchlorate, potassium manganate, potassium permanganate, potassium oxide, potassium hyperoxide, potassium silicate, potassium bromate, potassium iodate and the like; and organic potassium compounds typified by potassium formate, potassium acetate, potassium citrate, potassium oxalate, potassium fumarate, potassium butyrate, potassium lactate, potassium tartrate, potassium succinate, potassium oleate, potassium palmitate, potassium aspartate, potassium glutamate and the like.

Among these potassium compounds, inorganic potassium compounds are preferred, and potassium chloride is most preferred from the viewpoint of solubility in media and cytotoxicity.

The amount of the above potassium compound used is not particularly limited as long as the effects of the present invention are shown. For example, when a potassium compound used is potassium chloride, the amount can be commonly about 10 to 500 mM, and preferably about 50 to 200 mM.

The above ionophore is not particularly limited as long as the effects of the present invention are shown. Examples thereof can include Ionomycin, A23187 (Calcimycin), and 4-Bromo-A23187 and the like.

The amount of the above ionophore used is also not particularly limited as long as the effects of the present invention are shown. In the case of the above Ionomycin, for example, the amount can usually be about 1 µM.

The medium, culture conditions and the like to maintain eukaryotic cells expressing an olfactory receptor derived from a mammal; a site where the amount of calcium ions taken into eukaryotic cells expressing an olfactory receptor derived from a mammal is measured; and a means for measuring the amount of calcium ions taken into eukaryotic cells expressing an olfactory receptor derived from a mammal in step 2, can be the same as those illustrated in the above step 1.

### Step 3

Step 3 in the calculation method of the present invention is a step of calculating the ratio of the numerical value measured in step 1 and the numerical value measured in step 2. The ratio (proportion) calculated in step 3 can be a numerical value obtained by dividing the numerical value measured in step 1 by the numerical value calculated in step 2.

It should be noted that when the calculation methods in steps 1 and 2 use the above fluorescence value, a numerical value calculated in step 3 can also be more accurate by correcting a fluorescence value obtained using a prescribed concentration of chelating agent (e.g. EDTA or EGTA or the like) in a system in step1 and step 2 before step 3, with a background value.

The numerical value obtained in step 2 corresponds to the final event of a signal cascade caused in response to olfactory stimulation in cells expressing an olfactory receptor. In addition, the step of depolarizing cells in step 2 corresponds to a step of a positive control which is the final event of cells expressing an olfactory receptor to be measured in the calculation method of the present invention, and thus it is understood that a specific numerical value obtained by this is not easily affected by experimental conditions and the like and an absolute value to a certain extent is shown.

However, the numerical value obtained in step 1 varies depending on measurement conditions and the like, and is preferably obtained as a value relative to any reference value.

Therefore, by calculating a numerical value obtained in step 1 based on an absolute numerical value obtained in step 2, the degree of activation of cells expressing a specific olfactory receptor by a specific test substance can be calculated as an absolute numerical value to a certain extent.

### Calculation method (B) for degree of activation

A second method for calculating the degree of activation of a receptor by a test substance of the present invention will be illustrated. Specifically, the method includes the following steps 1 to 3.

### (Step 1)

Step of starting to measure fluorescence before a test substance is brought into contact with eukaryotic cells expressing a receptor to use the average value of fluorescence in a first prescribed time before the contact as Fbase.

### (Step 2)

Step of bringing the test substance into contact with eukaryotic cells expressing a receptor to use the greatest value of fluorescence in a second prescribed time after the contact with the test substance as Fmax.

### (Step 3)

Step of calculating Fmax/Fbase as an activation degree.

The present calculation method (B) for the degree of activation may be carried out in the order of step 1, step 2 and step 3. When repeating measurement, step 1, step 2 and step 3 may be carried out in this order, for example, in the second measurement too.

The above test substance is not particularly limited as long as the effects of the present invention are shown. A substance containing a component which is not generally recognized by the sensory organs of animals such as humans, for example, can also be included in the test substance in the calculation method of the present invention.

The category of the above test substance is not particularly limited as long as the effects of the present invention are shown. The test substance can be, for example, a pure substance or a mixture. In addition, the pure substance is not particularly limited to whether it is a single body or a compound.

The form of the above test substance is not particularly limited as long as the effects of the present invention are shown. The test substance may be in the form of, for example, a gas, a solid or a liquid.

The above receptor is not particularly limited as long as the effects of the present invention are shown. The receptor may be, for example, a prescribed receptor to which the nucleic acid 3 in the above-described array 1 belongs, indicates so-called transmembrane receptors, and can include, for example, metabotropic receptors, and ionotropic receptors.

Examples of metabotropic receptors are G protein-coupled receptors, tyrosine kinase receptors, and guanylyl cyclase receptors. Examples of G protein-coupled receptors are olfactory receptors, muscarinic acetylcholine receptors, adenosine receptors, adrenaline receptors, GABA receptors (type B), angiotensin receptors, cannabinoid receptors, cholecystokinin receptors, dopamine receptors, glucagon receptors, histamine receptors, opioid receptors, secretin receptors, serotonin receptors, gastrin receptors, P2Y receptors and rhodopsin. Examples of tyrosine kinase receptors are insulin receptors, cell growth factor receptors and cytokine receptors. Examples of guanylyl cyclase receptors are GC-A, GC-B and GC-C.

Examples of ionotropic receptors are nicotinic acetylcholine receptors, glycine receptors, GABA receptors (type A, type C), glutamic acid receptors, serotonin receptor type 3, inositol trisphosphate (IP3) receptors, ryanodine receptors and P2X receptors.

An existing substance taken into cells depending on the activation degree of these receptors can be a target to be measured. For example, when the receptor is an olfactory receptor, examples of intracellular substances can include calcium ion.

When the receptor is an olfactory receptor, a contact method with a test substance will now be illustrated. When the test substance is in the form of a solid, by applying a solution obtained using a known solvent which can elute an odor component included in the substance to eukaryotic cells, the test substance can be brought into contact with eukaryotic cells. In addition, when the test substance is in the form of gas, by applying a solution obtained using a known solvent which can absorb an odor component included in the test substance to eukaryotic cells, the test substance can be brought into contact with eukaryotic cells.

The above known solvent is not particularly limited as long as the solvent does not respond to an olfactory receptor derived from a mammal by the method of the present invention. Examples thereof can include water, a buffer solution, DMSO, methanol, ethanol, a medium, Ringer solution and the like.

### Step 1

Step 1 in the calculation method of the present invention is a step of measuring, when the test substance is brought into contact with eukaryotic cells expressing a receptor, the substance taken into the cells. A system in which the receptor is an olfactory receptor derived from a mammal and the substance taken into the cells is calcium ion will now be illustrated.

In step 1, the medium, culture conditions and the like to maintain eukaryotic cells expressing an olfactory receptor derived from a mammal are not particularly limited as long as they do not have an influence on the measurement of the amount of calcium ion taken into the eukaryotic cells in steps 1 and 2. Examples thereof can usually include media, temperatures and carbon dioxide concentrations in which cells derived from mammals can be cultured.

It should be noted that a site where calcium ion taken into eukaryotic cells expressing an olfactory receptor derived from a mammal is measured in step 1 is not particularly limited as long as the above medium and culture environment can be maintained. Examples thereof can include sites suitable for cell culture such as on a dish, a plate, a multiwell plate, a chamber and an array. From the viewpoint of carrying out the calculation method of the present invention with high throughput, measurement is preferably carried out on a multiwell, a chamber provided with a multiwell and an array. At this time, it is preferred that olfactory receptors derived from a mammal which are expressed by cells stored in each well, be different from each other.

The means for measuring calcium ion taken into eukaryotic cells expressing an olfactory receptor derived from a mammal in step 1 is not particularly limited as long as the effects of the present invention are shown. Examples thereof can include a means using a calcium ion-measuring fluorescent indicator and the like. The calcium ion-measuring fluorescent indicator has an effect in which fluorescence intensity varies depending on the amount of calcium ion.

The fluorescent indicator used in the above means is not particularly limited as long as the effects of the present invention are shown. For example, so-called calcium ion-measuring indicators to measure intracellular calcium ion can be used. For example, Calbryte 520, Calbryte 590 or Calbryte 630 is used, and changes in the concentration of intracellular calcium ion are colored as fluorescence by an indicator. These fluorescent indicators are preferably AM derivatives (protected with acetoxymethyl group) which increase cell membrane permeability.

It should be noted that because AM derivatives can be in the form of granules in water, for the purpose of allowing cells to take these in, a surface active agent such as Pluronic F-127 or Cremophor EL may be used. Among the above indicators, Calbryte 520 AM is preferably used from the viewpoint of being able to measure the amount of calcium ion taken into cells over time, being able to efficiently carry out measurement, and having characteristics of excitation at a single wavelength and fluorescence at a single wavelength, which are highly versatile around a microscope G excitation, and the like. A specific method for measuring the amount of calcium ion taken into cells using these pigments is not particularly limited. Specifically, examples thereof can include a method for measuring fluorescence values emitted by an indicator depending on the binding state of the indicator and intracellular calcium ion after being brought into contact.

(Step 1) is a step of starting to measure fluorescence before a test substance is brought into contact with eukaryotic cells expressing a receptor to use the average value of fluorescence in a first prescribed time before the contact as Fbase. Here, the first prescribed time before contact is a time for which a eukaryotic cell array 10 and measurement system measure thermally stable and chemically stable states before being brought into contact with a test substance. Therefore, the start time of the first prescribed time is set depending on time until thermal equilibrium after the eukaryotic cell array 10 is put on the measurement system. In addition, the finish time of the first prescribed time does not include a time when chemical equilibrium with an environment is lost, i.e. a time when a test substance is brought into contact with the eukaryotic cell array 10. Because the eukaryotic cell array 10 has a limited size and considering that a difference in time for the contact with a test substance arises on both the sides of the eukaryotic cell array 10, the finish time of the first prescribed time is desirably set before the contact with the test substance. The first prescribed time is for example a time from the onset of measurement to 1 minute before the contact with a test substance, and is typically for 5 minutes from 6 minutes to 1 minute before the contact with the test substance. The start time of the first prescribed time may be 30 minutes, 20 minutes, 10 minutes, 9 minutes, 8 minutes, 7 minutes or 5 minutes before the contact with a test substance, and the finish time of the first prescribed time may be 3 minutes, 2 minutes 30 seconds, 2 minutes, 1 minute 30 seconds, 50 seconds, 40 seconds, 30 seconds, 20 seconds or 10 seconds before the contact as long as the effects of the present invention are shown.

(Step 2) is a step of starting to measure fluorescence from around the time when a test substance is brought into contact with eukaryotic cells expressing a receptor to use the greatest value of fluorescence in a second prescribed time after the contact as Fmax. Therefore, it is required that the second prescribed time be set to include additional time in which maintenance of a tendency toward a reduction in fluorescence can be confirmed beyond Fmax (follow-up time). Here, the response speed of a test substance to a receptor is extremely fast, and thus it is desired that the start time of the second prescribed time be the same time as when the test substance is brought into contact with the receptor. On the other hand, as for the greatest value of fluorescence Fmax, the change in time from the peak to decline and the form of the downward curve vary depending on the types of receptor and test substances, and thus it is required that the additional time (follow-up time) be relatively long. As a result of investigations of many types of receptor and test substances, the peak value can be judged to be Fmax when a decline tendency is sustained for 200 seconds or more after fluorescence intensity is changed from the peak value to decline as a criterion. As another criterion, the peak value can be judged to be Fmax when a decline tendency is sustained over additional time, which is 0.3 times or more than the time elapsed from the start time of the second prescribed time to the peak value after the fluorescence intensity is changed from the peak value to decline. The second prescribed time is for example a time from 0 to 20 minutes after the contact with a test substance, and is typically for 10 minutes from 0 to 10 minutes after the contact with a test substance. The start time of the second prescribed time may be 3 seconds, 5 seconds, 10 seconds, 20 seconds or 30 seconds after the contact with a test substance, and the finish time of the second prescribed time may be 12 minutes, 15 minutes, 25 minutes or 30 minutes after the contact as long as the effects of the present invention are shown.

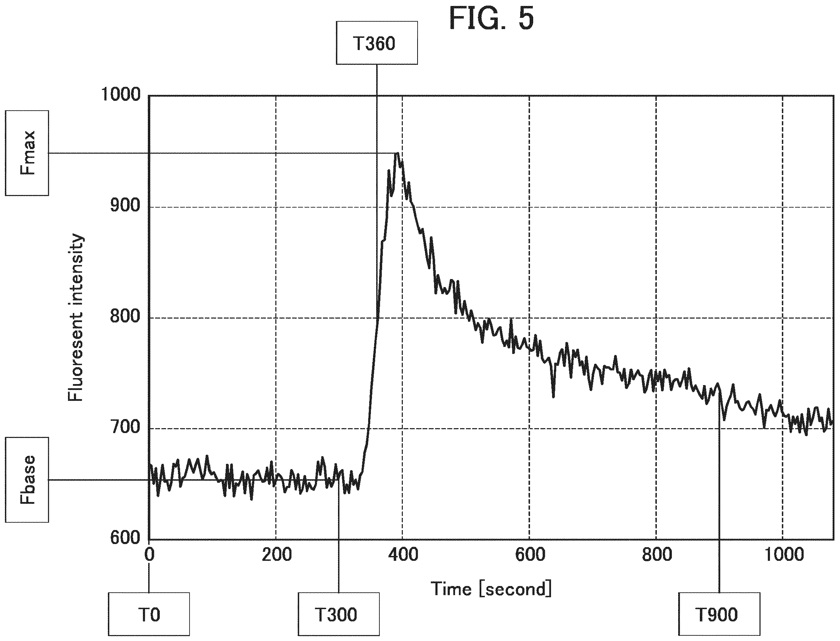

The activation degree of a receptor can be found by calculating Fmax/Fbase based on Fbase and Fmax thus found. Fig. 5 is a graph which shows an example of fluorescent measurement by the calculation method (B). Time (sec) is taken along the abscissa and fluorescence intensity (arbitrary unit) is taken along the ordinate. The onset of measurement is 0 sec in the time on the abscissa, and a test substance is brought into contact with eukaryotic cells expressing a receptor at 360 seconds after the onset of measurement (shown by T360 in Fig. 5). In a case in Fig. 5, the first prescribed time is from T0 to T300, that is for 5 minutes from 6 minutes to 1 minute before the contact with a test substance. In addition, the second prescribed time is from T360 to T900, that is for 10 minutes from 0 minutes to 10 minutes after the contact with a test substance.

As shown in Fig. 5, fluorescence in the first prescribed time is stable, and the average value thereof is suitable as a base line. In addition, fluorescence in the second prescribed time has a sharp peak, and it is easy to detect the greatest value. Therefore, a value obtained by dividing Fmax by Fbase can be an indicator to effectively express an activation degree. Because Fbase = 656 and Fmax = 949 in the case in Fig. 5, based on the numerical values, the activation degree is calculated as Fmax/Fbase = 1.4.

As an advantage the calculation method (B) has high measurement reproducibility in which the depolarization step and contact step of a positive control substance are not necessarily needed, compared to the calculation method (A).

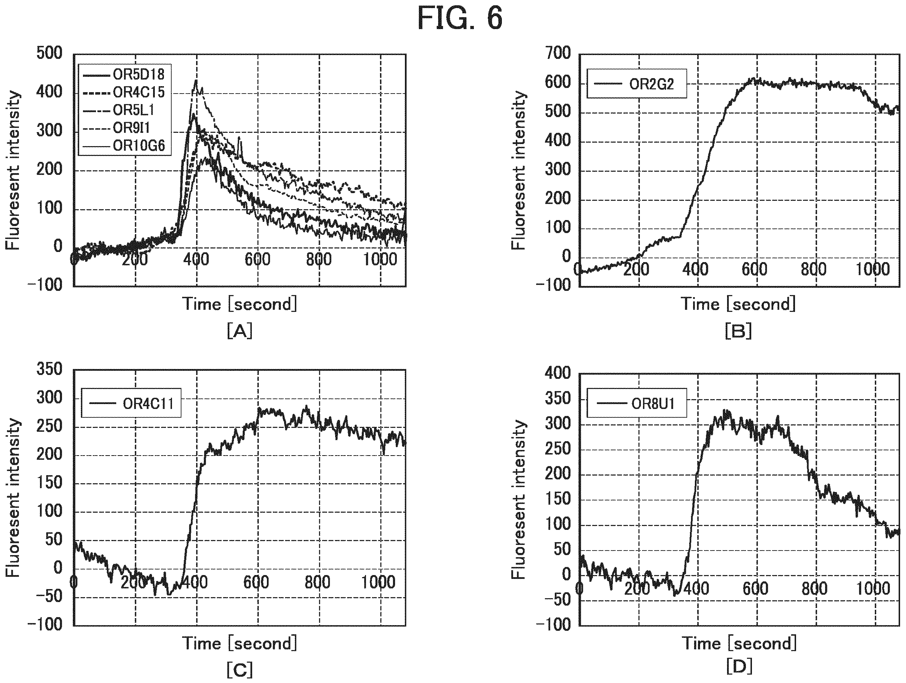

In Figs. 6 the temporal response of fluorescence intensity when a test substance is brought into contact with a variety of transiently expressed olfactory receptors is measured (a test substance has been brought into contact with them from the onset of measurement to 360 seconds). Fig. 6[A] shows results obtained by examining the temporal response of 5 typical examples of olfactory receptors. As shown in Fig. 6[A], fluorescence intensity shows the greatest value at 400 to 450 seconds after the onset of measurement and then decays. Regarding a receptor OR8U1 (Fig. 6[D]), however, fluorescence shows the greatest value around 500 seconds after the onset of measurement and then decays. In addition, regarding a receptor OR2G2 (Fig. 6[B]) and a receptor OR4C11 (Fig. 6[C]), fluorescence shows the greatest value around 600 seconds after the onset of measurement and then decays after a period of almost flat from 600 to 900 seconds. As described above, temporal response varies depending on receptors, and some receptors are affected by the contact with a test substance for a prolonged time. In addition, tendencies of fluorescence intensity before the onset of measurement are different depending on receptors.

Because of this it is desirable that the second prescribed time be set to be longer than the first prescribed time, and as long as such a time is set, a measurement value Fmax/Fbase has high reliability as an activation degree indicator. As long as such a measurement method is used, activation degrees can be repeatedly measured by repeated contact with a test substance without contact with a positive control substance. Typically the first prescribed time is for 5 minutes from 6 minutes to 1 minute before the contact with a test substance, and the second prescribed time is for 10 minutes from 0 minutes to 10 minutes after the contact with a test substance.

It should be noted that the reliability of the calculation method (B) can also be further improved by using a means for quality control at the same time. For example, disturbance is mixed into a whole measurement system and noises can be superimposed on measurement signals (fluorescence intensity), but the reason is unclear. In addition, fluorescence intensity can be reduced as the whole measurement system. Even in measurement in such cases, as a method for securing reliability, the standard deviation (SD) of fluorescence intensity in the first prescribed time is measured and used as a parameter to judge whether or not to use it as an activation degree. Specifically, Fbase + 5 SD is calculated, and is compared to the above-described measurement value Fmax. When the measurement value Fmax is not less than Fbase + 5 SD, it is used as an activation degree, and when the measurement value Fmax is less than Fbase + 5 SD, it is not used as an activation degree. Reliability when using a measurement value Fmax/Fbase as an activation degree is further improved by statistically judging the measurement value as described above. In the case in Fig. 5, for example, the standard deviation (SD) of fluorescence intensity in the first prescribed time is SD = 9, and Fbase + 5 SD = 701 is calculated because Fbase = 656 and Fmax = 949 as described above. At this time, Fmax is not less than Fbase + 5 SD, and can be sufficiently used as an activation degree.

As described above, the measurement value Fmax/Fbase originally has high reliability as an activation degree indicator, and thus the activation degree can be repeatedly measured by repeated contact with a test substance without the contact with a positive control substance. When the measurement value is required to have high reliability in this repeated measurement, the second measurement following the first measurement can be carried out without the influence of the first measurement by leaving the system until Fbase in the second measurement (hereinafter can be referred to as Fbase 2) is below the above Fbase + 5 SD (for example, perfusing Ringer solution). The time when Fbase 2 < Fbase + 5 SD is obtained (relaxation time) varies depending on receptors and test substances and is typically 20 minutes or more. Similarly, the third and subsequent measurements can be also carried out using this method.

On the other hand, when measurement is repeatedly carried out in an array in which a plurality of receptors are arranged, because relaxation time varies depending on receptors, a lot of time can be required when leaving the system until all the receptors meet the conditions of Fbase 2 < Fbase + 5 SD. Therefore, when time efficiency of measurement is a priority, even in a time of Fbase 2 ≥ Fbase + 5 SD, the second measurement can be carried out. That is, the second measurement can be continuously carried out by carrying out step 1 to find Fbase 2 before the second measurement. Similarly, the third and subsequent measurements can be carried out.

The calculation method (B) for activation degrees of the present invention has been illustrated using a system in which a substance taken into eukaryotic cells by contact with a test substance is detected using a fluorescent indicator as a typical example; however, the calculation method (B) is not limited thereto, and can be widely applied. For example, the calculation method (B) can be applied to a system in which ions taken into eukaryotic cells by contact with a test substance are detected using an ion measuring fluorescent pigment or a fluorescent binding protein in the same manner.

In addition, the calculation method (A) for activation degrees of the present invention and the calculation method (B) for activation degrees of the present invention can also be applied to a eukaryotic cell array 10 of the present invention, particularly a eukaryotic cell array in which a receptor has been expressed (can be referred to as receptor array 10r in the description).

It should be noted that the activation degree can be expressed as another numerical value by means of a numerical value calculation based on Fmax/Fbase. For example, when the greatest activation degree of a receptor is considered to be 100%, the activation degree can be expressed as a percentage (%) with respect to this. Specifically, the greatest activation degree of a receptor can be found by treatment using a positive control reagent such as, for example, a calcium ionophore (a reagent to increase the concentration of intracellular calcium ion to 100%) or dbcAMP (a reagent to activate CNG of calcium channel to 100%) in place of a test substance. In the case in the above Fig. 5, Fmax/Fbase in the greatest activation degree has already been found to be 2.2, and thus activation by a test substance in a measurement value of Fmax/Fbase = 1.4 in Fig. 5 can also be expressed as 1.4/2.2 = 0.64, that is, 64% of the greatest activation degree of the receptor has been shown.

### Method for measuring receptor array

A method for measuring the activation degree of each receptor in the receptor array 10r of the present invention is typically as follows. Here, the method will be illustrated by a case using the calculation method (B) for activation degrees. A measuring fluorescent substance, measuring fluorescent pigment or fluorescent binding protein is allowed to act on a receptor array 10r to establish a reaction system in which an activation degree by contact with a test substance is replaced with fluorescence. Subsequently, a measurement system is established to collectively acquire a region including all through-holes 2 of the receptor array 10r as an image data at the same time. In the measurement system, a light receiving optical system is set so that not only the through-holes 2 (through-hole 2A, etc.) of the receptor array 10r will be individually recognized but also each eukaryotic cell (eukaryotic cells 4A1, 4A2, 4A3, 4A4, 4A5, etc.) in the interior of the through-hole 2 can be individually recognized. Subsequently, a test substance is brought into contact with the receptor array 10r in which the reaction system has been established. Here, the measurement system is operated before the contact with the test substance, and image data are acquired during a first prescribed time. In addition, the measurement system is operated around the time of the contact with the test substance, and image data are acquired during a second prescribed time. The fluorescence of each eukaryotic cell is obtained as a numerical value after a lapse of the second prescribed time, and the activation degree is calculated by an operation of numerical values on a cell basis. At this time, the numerical values of fluorescence on a cell basis are quality controlled by a means for setting a slice level (a fixed value of fluorescence intensity), only data not less than a prescribed value are used, and it is also acceptable to calculate an activation degree by an operation of numerical values on a cell basis. In addition, the activation degree on a cell basis is quality controlled by a judgement using Fbase + 5 SD, and it is also acceptable to use and read out data with high reliability. In addition, the activation degree on a cell basis is quality controlled by a known statistical means, and it is also acceptable to calculate and read out one data per receptor. As a result, for example, it is acceptable to read out numerical values of 400 activation degrees of 400 receptors.

The numerical values calculated by the method of the present invention can be used as a group of information, for example, with a receptor taken as a calculation target of this numerical value, the concentration of a test substance allowed to act on the receptor, and the like. Therefore, when the receptor is an olfactory receptor, a group of information including the numerical values calculated by the present invention is used as a database, and is useful in a method for forming an odor of the present invention or a method for producing a composition having a formed odor as described below, or the like.

The numerical values obtained by the calculation method of the present invention can be used for the various uses described below.

For example, it is possible to provide a method for reproducing an odor of a substance by combining odors of another two or more substances. This will be described in a method for forming an odor of the present invention below in detail. It is also possible to provide a method for reproducing a composition having a formed odor of a substance, by combining another two or more substances. This will be described in a method for producing a composition having a formed odor below in detail.

### Method for forming an odor

The method for forming an odor of a target substance of the present invention (hereinafter this can be referred to as "forming method" in the description) is a method for forming an odor of a target substance by combining two or more reference substances so that the degree of activation of each olfactory receptor derived from a mammal by the target substance is formed based on the degree of activation of each olfactory receptor derived from a mammal by the two or more reference substances.

The means for getting the above degree of activation of each olfactory receptor derived from a mammal by two or more reference substances is not particularly limited as long as the effects of the present invention are shown. For example, numerical values obtained by the calculation method (A) and/or calculation method (B) of the present invention (A) can be used.

The means for getting the above degree of activation of each olfactory receptor derived from a mammal by a target substance is not particularly limited as long as the effects of the present invention are shown. For example, numerical values obtained by the calculation method (A) and/or calculation method (B) of the present invention (A) can be used.

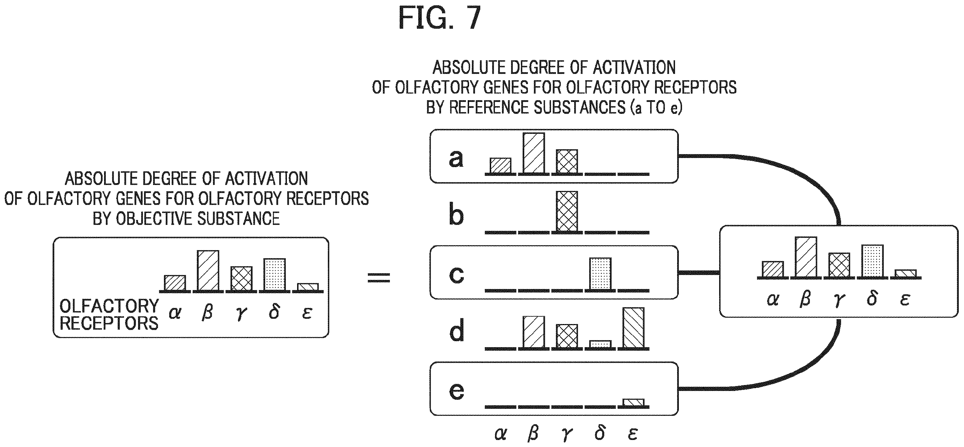

A specific combination method will be illustrated based on the schematic diagram shown in Fig. 7. The schematic diagram in Fig. 7 shows that an odor of a target substance is formed, that is, it shows the degree of activation of olfactory receptors (e.g. a, b, g, d and e) derived from a mammal by an objective substance.

The schematic diagram in Fig. 7 shows that the degree of activation of olfactory receptors derived from a mammal by a target substance can be formed by combining, for example, a, c and e of reference substances a, b, c, d and e. That is, the combination method is a pattern matching method, and the activation degree of olfactory receptors a, b, g, d and e by a target substance, and the activation degree of olfactory receptors a, b, g, d and e by each reference substance are compared and the closest substances are selected. In many cases, a target substance cannot be formed from one reference substance. Therefore, this pattern matching method is carried out several times. In the case of Fig. 7, for example, if a reference substance a is selected by a first pattern matching method, receptors a, b and g will be achieved. Therefore, the second pattern matching method is carried out focusing on only receptors d and e. As a result, a reference substance c is selected, and the third pattern matching is carried out focusing on only a receptor e in the same manner. As a result, a reference substance e is selected.

It should be noted that when in regard to the degree of activation of an olfactory receptor derived from a mammal by the above reference substances, linearity is not observed between the stimulation concentration (test substance concentration) of the olfactory receptor derived from a mammal by the reference substance and the degree of activation of the olfactory receptor derived from a mammal which is shown in the event of a reaction with the substance, a combination can be made so that the degree of activation of the olfactory receptor derived from a mammal by a target substance will be formed based on the degree of activation of the olfactory receptor derived from a mammal by a specific concentration of reference substances.

It should be noted that non-linearity is observed in many cases between the stimulation concentration (test substance concentration) of an olfactory receptor and the activation degree of the olfactory receptor, and a relationship between these cannot be expressed by function in some cases. In such cases, it is thought that data on the activation degree of various olfactory receptors by a reference substance with a plurality of concentrations is collected and this operation is carried out using a further plurality of reference substances to obtain numerical values (obtain the data). The data thus obtained (hereinafter can be referred to as first data) can be expressed by a three dimensional matrix of reference substance(s), concentration(s) and receptor(s). In addition, the second data can be obtained by the same operation for obtaining numerical values using a target substance, and can be expressed by a one dimensional matrix. Therefore, an optimal solution closest to the target substance can be obtained from the combination of a plurality of reference substance candidates and a plurality of concentration candidates by a prescribed operation of the first data using the second data. In addition, an examination step of performing an examination by finding a difference between a virtual substance formed using a plurality of reference substances (computational virtual formed substance) and a target substance may be provided in the course of operation. Method for producing a composition having a formed odor

The method for producing a composition having a formed odor of a target substance of the present invention is a method for producing a composition having a formed odor of the target substance by combining two or more reference substances so that the degree of activation of each olfactory receptor derived from a mammal by the target substance is formed based on the degree of activation of each olfactory receptor derived from a mammal by the two or more reference substances.

The means for getting the above degree of activation of each olfactory receptor derived from a mammal by two or more reference substances is not particularly limited as long as the effects of the present invention are shown. For example, numerical values obtained by the calculation method of the present invention can be used.

The means for getting the above degree of activation of each olfactory receptor derived from a mammal by a target substance is not particularly limited as long as the effects of the present invention are shown. For example, numerical values obtained by the calculation method of the present invention can be used.

A specific combination method can be illustrated based on the schematic diagram shown in Fig. 7 as illustrated in the above method for forming an odor. That is, in the case of Fig. 7, a target substance can be formed by mixing reference substances a, c and e in an equal amount.

As for a composition produced by the production method of the present invention, even if a target substance is expensive, a composition having a formed odor equal to that of the target substance can be made inexpensively.

### Method for screening substance to modify sample odor into objective odor

An odor can be modified by applying the above-described method for forming an odor. That is, the activation degree of olfactory receptors (e.g. a, b, g, d and e) is measured using both a sample odor to be modified and an objective odor. Here, the above-described calculation method (A) and/or calculation method (B), for example, can be used to evaluate activation degrees.

Here, an objective odor and a sample odor to be modified are compared in each receptor. Subsequently the type and amount of reference substance required for a specific receptor (e.g. receptor a) are investigated to make an odor close to one detected by the target receptor a, and candidate substances to be added are selected from a plurality of reference substances. At this time, in regard to an odor to be modified and an odor to which a selected reference substance has been added, selection is carried out based on the criteria of whether or not the activation degree of the receptor is close to the activation degree of the receptor a of a target odor. Adding odors may be actually mixing and forming reference substances, or computational virtual constitution. A judgement as to whether or not an odor is getting close to a target odor is made based on the activation degree of the receptor a found by a mixed and formed substance. Such an operation is repeatedly carried out on the receptor a, and candidate substances can be selected when an odor is closest to the target odor. For ease of explanation, the receptor a has been focused on; however, such an operation can be simultaneously carried out on a plurality of receptors. When the types of receptor are n types, for example, candidate substances can be selected so that Euclidean distance in an n dimensional space is smallest.

In addition, existing information about the activation degree of each receptor by a reference substance is held in a database, and selection may be carried out based on the criteria of whether or not the activation degree of the receptor is getting close to the activation degree by a target odor by comparison with information in the database. In addition, the database is illustrated above as a database of substances emitting typical odors; however, in addition to this, the database may be a database of substances eliminating typical odors.

The method for modifying a sample odor into an objective odor can be applied to modify an odor in an environment (space) into a comfortable one, and also to eliminate (deodorize) an odor in an environment (space) and modify the odor into a comfortable one. It is also possible to provide a deodorant substance as a further application of the screening method.

### EXAMPLES

Examples will now be described to illustrate the present invention in more detail. It is needless to say that the present invention is not limited to the following examples.

### 1) Isolation of mouse olfactory receptor

C57/BL6J (male) aged 3 to 5 weeks were purchased from Japan SLC, Inc. The mice were beheaded after intravenous anesthesia with 3 mL of Urethane solution (300 mg/mL; U2500, Sigma-Aldrich) and olfactory epithelium was extracted on ice. The extracted olfactory epithelium was dispersed in a Ca²⁺ ringer solution (pH 7.2; 140 mM NaCl, 5 mM KCl, 1 mM CaCl₂, 1 mM MgCl₂, 10 mM HEPES, 10 mM glucose, and 1 mM sodium pyruvate) containing 1 mM Cystein, and treated with 1 unit/mL papain or 0.25 mg/mL trypsin. An equal amount of Ca²⁺ ringer solution containing 0.1 mg/mL DNase I, 0.1 mg/mL BSA, and 500 mM leupeptin (0.025 mg/mL trypsin inhibitor in the case of trypsin) was added thereto to stop the enzyme reaction, and the obtained solution then allowed to pass through a cell strainer twice to remove large tissue fragments.

The sample after removal was subjected to centrifugation treatment, and this was washed once to obtain an olfactory epithelium-derived cell solution.

### 2) Use of OneCell system and analytical means using the same

The olfactory epithelium-derived cell solution obtained above was subjected to centrifugation at 1000 rpm for 5 minutes (2420, KUBOTA Co.) to remove the supernatant, and to this, a Ca²⁺ ringer solution containing 250 mL of 5 mM Fluo4-AM (F312, Dojindo) and 0.02 wt% Pluronic F-127 (P6867, Molecular Probes) was then added. A polystyrene slide having about 400000 nano chambers with a diameter of 10 mm for As OneCell Picking system (AS ONE Corporation; automated single-cell analysis and isolation system [equipped with temperature control system, time lapse system, perfusion system]; Yoshimoto, et al., Scientific Reports Vol. 3 (2013) 1191; hereinafter this can be referred to as "OneCell system" in the description) was immersed in 2% PVP (polyvinyl pyrrolidone (NACALAI TESQUE, INC.)) and then put on a perfusion jig for the OneCell system. The above treated olfactory epithelium-derived cell solution was added thereto, and light centrifugation (7 x g, 1 min, three times) was carried out to store olfactory epithelium-derived cells in the nano chambers.

Subsequently, the slide was put on a prescribed site of the OneCell system and maintained with a Ca²⁺ ringer solution having been circulated, and fluorescent images derived from Fluo-4 of each olfactory epithelium-derived cell included in the nano chambers were observed through 4 x objective lens, and changes in fluorescence intensity over time were recorded.

The olfactory epithelium-derived cells were stimulated as follows. A Ca²⁺ ringer solution was refluxed to the slide in which the above olfactory epithelium-derived cells had been stored for 2 minutes (250 mL/min) and 100 mL of high K⁺ ringer solution (pH 7.2; 140 mM NaCl, 100 mM KCl, 1 mM CaCl₂, 1 mM MgCl₂, 10 mM HEPES, 10 mM glucose, and 1 mM sodium pyruvate) was then refluxed for 20 seconds. The site of olfactory sensory neurons included in olfactory epithelium-derived cells was confirmed by an increase in transient fluorescence derived from Fluo-4. As a result, the number of olfactory sensory neurons of olfactory epithelium-derived cells stored in the slide was about 10000.

### 3) Identification of olfactory genes

The olfactory epithelium-derived cells were stimulated by an odor molecule, 2-pentanone (30 mM) for 10 seconds and 2-pentanone (3 mM) for 10 seconds under the above conditions, and olfactory sensory neurons included in olfactory epithelium-derived cells were then identified by a high K⁺ ringer solution. As a result, approximately 100 of approximately 10000 olfactory sensory neurons reacted with 2-pentanone. Therefore an arbitrary 2 cells of these olfactory sensory neurons (cell number HI28-03 and HI25-18) were collected on a cell basis (volume 50 nL) by a glass capillary provided with the OneCell system and transferred to a PCR tube including 4.5 mL of Cell lysis solution (Cell Amp Whole Transcriptome Amplification Kit Ver. 2; Takara). After that, a reverse transcription reaction was immediately carried out in accordance with the instructions of the kit.

Next, a product of the reverse transcription reaction was subjected to PCR using a forward primer (SEQ ID NO:10) for the third transmembrane domain in the mouse olfactory receptor and a reverse primer (SEQ ID NO:11) or (SEQ ID NO:12) for the seventh transmembrane domain.

LA-Taq (Takara) was used as an enzyme for PCR and GC buffer I (Takara) was used as a PCR buffer, and after 1 cycle of 94°C 1 min, 35 cycles of 94°C 0.5 min, 40°C 0.5 min and 72°C 2 min were carried out, and finally 1 cycle of 72°C 5 min was carried out. A DNA fragment thus obtained was sub-cloned into pMD20 plasmid (Takara), and the base sequences of olfactory receptors expressed in cells (HI28-03 and HI25-18) which had reacted with 2-pentanone were identified.

As a result, Olfr168 (mOR271-1; GenBank accession number: AY317252) and Olfr205 (mOR182-11P; GenBank accession number: BC150839) were identified in HI28-03 and HI25-18 respectively.

By the above means, two olfactory sensory neurons (cell number IG04-13 and HG28-24) which had responded to pyridine as an odor molecule were obtained from olfactory sensory neurons included in olfactory epithelium-derived cells. Olfactory receptors expressed in these cells were identified by the above method; accordingly, Olfr45 (mOR253-2; GenBank accession number: AY317653) was identified in IG04-13 and Olfr166 (mOR270-1; GenBank accession number: AY317250) was identified in HG28-24.

An olfactory sensory neuron (cell number HE22-23) which had responded to 2-butanone as an odor molecule was obtained from olfactory sensory neurons included in olfactory epithelium-derived cells by the above means. An olfactory receptor expressed in this cell was identified by the above method; accordingly, an olfactory receptor Olfr1258 (mOR232-3; GenBank accession number: AY318460) was identified.

### 4) Calculation method for degree of activation of identified olfactory genes (calculation method (A))

HI28-03 cells and HI25-18 cells which had responded to stimulation by 2-pentanone of the above olfactory sensory neurons were stimulated by 2-pentanone again, and the amount of Ca²⁺ ion taken after stimulation to these cells was measured as an increased amount of fluorescence intensity derived from Fluo-4.

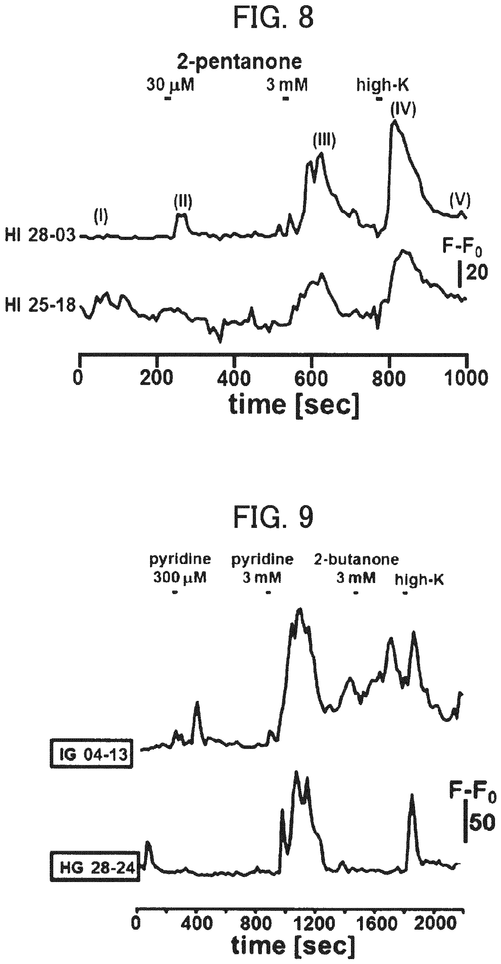

Specifically, the above Ca²⁺ ringer solution was refluxed to the slide glass which had been put on the OneCell device and had stored olfactory epithelium-derived cells, and a Ca²⁺ ringer solution containing 3 mM 2-pentanone described below was added thereto for 20 seconds every two minutes. The degree of increase in transient fluorescence intensity derived from Fluo-4 in each olfactory sensory neuron was repeatedly measured 5 times (Fig. 8).

As a result, the fluorescence intensity was increased to 46 ± 12 in HI28-03 cells and to 38 ± 10 in HI25-18 cells. The CV values of these numerical values, however, were 26% in both the cells, which showed unstable measurement.

Therefore, when an increased value of fluorescence intensity derived from Fluo-4 obtained by depolarizing the CNG channel of HI28-03 cells and HI25-18 cells, which had responded to 2-pentanone, by the above high K⁺ ringer solution was considered 100% and an increased value of fluorescence intensity derived from Fluo-4 of both the olfactory sensory neurons after stimulation by various concentrations of 2-pentanone was expressed by a relative value to the above numerical value (the experiment was independently carried out 5 times), the relative values were 74 ± 3 in HI28-03 cells and 67 ± 4 in HI25-18 cells, whose CV values were 4% and 6% respectively, which were significantly stable.

In addition, IG04-13 cells and HG28-24 cells which had responded to stimulation by pyridine of the above olfactory sensory neurons were stimulated by pyridine again, and the amount of Ca²⁺ ion taken after stimulation to these cells was measured as an increased amount of fluorescence intensity derived from Fluo-4 (Fig. 9).

As a result, the fluorescence intensity was increased to 195 ± 70 in IG04-13 cells and 153 ± 47 in HG28-24 cells. The CV values of these numerical values, however, were 36% and 31% respectively, which was a very unstable measurement.

Therefore, when an increased value of fluorescence intensity derived from Fluo-4 obtained by depolarizing the CNG channel of IG04-13 cells and HI28-24 cells, which had responded to pyridine, by the above high K⁺ ringer solution was considered 100% and an increased value of fluorescence intensity derived from Fluo-4 of both the olfactory sensory neurons after stimulation by various concentrations of pyridine was expressed by a relative value to the above numerical value (the experiment was independently carried out 5 times), the relative values were 120 ± 6 in IG04-13 cells and 132 ± 5 in HG28-24 cells, whose CV values were 5% and 4% respectively, which were significantly stable.

It is verified that high quantitativity of the above measurement system is maintained in the measurement of the amount of changes in fluorescence intensity derived from Fluo-4 based on not only peak height but also peak area. It is verified that as a depolarizing agent for the CNG channel existing in olfactory sensory neurons expressing olfactory receptors, not only high K⁺ ringer solution but also an existing ionophore can be used.

### 5) Reconstruction experiment

In order to verify whether or not the mouse-derived olfactory receptors obtained in the above 3) and 4) surely respond to odor molecules used for screening, a system in which adenylate cyclase is activated from a olfactory receptor through heterotrimeric G protein including Gaolf to accumulate cAMP from ATP, and luciferase expression is induced in an intracellular cAMP concentration-dependent manner, was reconstructed in HEK 293 cells.

Specifically, each of mouse-derived olfactory receptor genes, Olfr168, Olfr205, Olfr45 and Olfr166, and DNA coding for the amino acid sequence shown in SEQ ID NO:9 were fused. The obtained genes were incorporated into pME18S plasmid incorporated into downstream of SRa promoter, a plasmid having a base sequence coding for the amino acid sequence shown in SEQ ID NO:2 to allow Gaolf to be expressed, a plasmid to allow Receptor-transporting protein 1S (RTP1S) having a base sequence shown in SEQ ID NO:6, which is a factor to raise the efficiency of presentation of olfactory receptors from the Golgi body to the cell surface, to be expressed, and a cAMP-responsive luciferase expressing plasmid pGlosensor-22 (Promega Corporation). The plasmids are introduced into HEK 293T cells by Lipofectamine 2000 (Life Technologies) to cause transient expression.

Because of this, in a case where a mouse-derived olfactory receptor gene, Olfr168, was expressed, when luciferase activity induced by stimulation of an odor molecule 2-pentanone diluted in stages was measured, the luciferase activity increased in a 2-pentanone concentration-dependent manner, and the EC50 value was calculated to be 2.1 ± 0.6 mM. This is responsiveness to 2-pentanone similar to that of an olfactory sensory neuron expressing Olfr168 (Fig. 8), which shows that one of the odor molecules which can activate Olfr168 is 2-pentanone. In addition, the EC50 value (about 4 mM) of HEK293 cells expressing Olfr205 shows responsiveness to 2-pentanone similar to that of an olfactory sensory neuron expressing Olfr205, which shows that one of the odor molecules which can activate Olfr205 is 2-pentanone. Next, the EC50 value (about 5 mM) of HEK293 cells expressing Olfr45 or Olfr166 shows responsiveness to pyridine similar to that of an olfactory sensory neuron expressing Olfr45 or Olfr166, which shows that one of the odor molecules which can activate Olfr45 or Olfr166 is pyridine. Furthermore, the EC50 value (about 1.5 mM) of HEK293 cells expressing Olfr1258 shows responsiveness to 2-butanone similar to that of an olfactory sensory neuron expressing Olfr1258, which shows that one of the odor molecules which can activate Olfr1258 is 2-butanone.

As described above, it was found that each olfactory sensory neuron expressing a group of olfactory receptors corresponding to arbitrary odor molecules could be isolated by the method described in the above 3).

### 6) Production of human olfactory receptor-expressing plasmid

Plasmids each expressing 404 types of human-derived olfactory receptor gene selected from a group of human-derived olfactory receptor genes are constructed by synthetic DNA. A gene coding for amino acids shown in SEQ ID NO:X9 is fused to each of the above human-derived olfactory receptor DNA, and these are expressed by SRa promotor.

Furthermore, in order to make the expression level of human-derived olfactory receptor per cell constant, a plasmid (pcDNA5/FRT) supporting Flp-In system (Invitrogen), in which a gene is inserted only in a fixed site on the chromosomes of HEK293 cells and CHO cells is used.

### 7) Production of expression plasmid of factor group to support expression of human olfactory receptor

In order to have human-derived olfactory receptor genes be efficiently expressed in mammalian cells with the function maintained, a mammalian cell-expressing plasmid including DNA (SEQ ID NO:5) coding for Receptor-transporting protein 1 (RTP1), which is thought to be a factor to raise the efficiency of presentation of a human-derived olfactory receptor from the Golgi body to cell surface, or RTP1S (SEQ ID NO:6), which is a transcript variant thereof, DNA (SEQ ID NO:7) coding for Receptor-transporting protein 2 (RTP2) and DNA (SEQ ID NO:8) coding for REEP1 is produced.

Specifically, the above DNAs (RTP1, RTP2, REEP1 and blasticidin-resistant (Bsr) gene, which is a drug selection marker) are tandemly inserted between a hybrid promoter of Human Elongation Factor-la (EF-1a) promoter, which is not easily affected by epigenetic effects, and the R segment and part of the U5 sequence (R-U5') derived from Human T-cell Leukemia Virus 1 (HTLV1)-derived LTR, and the SV40-derived poly A site in the multicloning site of pUC18 plasmid in accordance with Kozak's rule to produce a mammalian cell-expressing plasmid, named Construct 1.

### 8) Production of Gaolf and CNG-expressing plasmid for human-derived olfactory receptor-expressing cells

Even when a mammal-derived olfactory receptor is ectopically expressed in cells in the method described in the above 5) reconstruction experiment, an increase in the intracellular cAMP concentration has been used as an indicator when measuring activation by an odor molecule. However, this method is an end point assay, and is not a real time assay which can exhaustively analyze a cell group expressing human-derived olfactory receptors with high throughput.

There currently exist some fluorescent proteins such as Flamindo 2 in which fluorescence intensity is changed in a cAMP concentration-dependent manner; however, the dynamic range of cAMP concentration to which most of them can respond is narrow, or fluorescence decays with an increase in cAMP concentration. This is inconvenient when attempting to instantly select cells with an increased concentration of cAMP from a lot of cell groups, or observation cannot be carried out by excitation at a wavelength and fluorescence at a wavelength, which are suitable for instant measurement. Therefore, there does not exist a fluorescence detection system in which intracellular cAMP can be measured on real time with high sensitivity. It is thus required to allow cyclic nucleotide gated ion-channel (CNG) to be expressed at the same time to achieve real time analysis for a cell group expressing human-derived olfactory receptors.

Therefore, the expression of three types of gene forming CNG (CNGA2 subunit, CNGA4 subunit, CNGB1b subunit) is attempted. As the DNA used, DNA including a base sequence shown in SEQ ID NO:1, SEQ ID NO:3 and SEQ ID NO:4 is used for CNGA2 subunit, CNGA4 subunit, and CNGB1b subunit respectively.

Specifically, synthetic DNA constructed from CNGA2 subunit gene in accordance with Kozak's rule, SV40 poly A site, hEF1-HTLV promoter, and Gaolf gene in accordance with Kozak's rule is tandemly inserted into the multicloning site between the CMV promoter of pcDNA3.1⁺/C-(K) DYK plasmid having a neomycin-resistant gene and SV40 poly A site, and this is named Construct 2.

Next, when using mutants (E342G/C460W/E583M) of three amino acid residues of CNGA2 subunit, which are not easily depolarized (the 342^{nd} glutamic acid, 460^{th} cysteine, and 583^{rd} glutamic acid in an amino acid sequence coded by a base sequence shown in SEQ ID NO:1), a decay in the concentration of intracellular Ca²⁺ is inhibited. Therefore, even an endpoint assay is suitable when efficiency of activation of human-derived olfactory receptors is weak.

Specifically, CNGA2 subunit is subjected to the above mutation, and an expression plasmid is produced in the same manner as in Construct 2 (this is named Construct M2).

In order to measure an increase in the concentration of intracellular Ca²⁺ in actual measurement, measurement can be carried out using only CNGA2 subunit in the case of CNG subunit, and sufficient sensitivity can be obtained by Construct 2 and Construct M2. However, CNGA4 subunit and CNGBlb subunit can be also expressed to further sufficiently show the activity of CNG.

Therefore, genes (CNGA4 subunit gene and CNGBlb subunit gene and puromycin-resistant (Puro) gene, which is a drug selection marker) are tandemly inserted between hEF1-HTLV promoter and SV40-derived poly A site in the multicloning site of pUC18 plasmid by Kozak's rule, and this is named Construct 3.

### 9) Incorporation into HEK293 cells and CHO cells

HEK293 cells or CHO cells are transformed using Construct 1 by a conventional method, and selection is carried out by blasticidin to obtain cells which stably retain blasticidin-resistant Construct 1 and express RTP1, RTP2 and REEP1. Next, the above transformed cells are transformed using Construct 2, and selection is carried out by neomycin to obtain cells which stably retain neomycin and blasticidin-resistant Construct 1/2 and express CNGA2 subunit and Gaolf in addition to RTP1, RTP2 and REEP1. These are named HEK293-C1/2 strain and CHO-C1/2 strain respectively.

Furthermore, both the strains are transformed using Construct 3, and selection is carried out by puromycin to obtain cells which stably retain neomycin, blasticidin and puromycin-resistant Construct 1/2/3 and express RTP1, RTP2, REEP1, CNGA2 subunit, Gaolf, CNGA4 subunit and CNGBlb subunit. These are named HEK293-C1/2/3 strain and CHO-C1/2/3 strain respectively.

Here, for the purpose of incorporating a vector expressing a human-derived olfactory receptor into a fixed site on the chromosome of cells, an expression strain which stably retains the above Construct 1/2 and Construct 1/2/3 is produced in the same manner using Flp-In-293 cells and Flp-In-CHO cells (both the cells are manufactured by Invitrogen) in place of the above HEK293 cells or CHO cells. These are named F293-C1/2; FCHO-C1/2; F293-C1/2/3; FCHO-C1/2/3 respectively. 10) Isolation of human olfactory receptors which respond to an arbitrary odor

The above HEK293-C1/2 strain is transformed using 404 types of the human-derived olfactory receptor-expressing plasmid produced in the above 6), and the obtained strains are loaded in the OneCell machine by the method in 2). When the strains are stimulated by 10 mM of an optional odor molecule (vanillin) in the method in 3), it is expected that an increase in fluorescence intensity is observed in some cells. Therefore, these cells are collected, and PCR is carried out to amplify a human-derived olfactory receptor gene site inserted into pME18S. By analysis, the amplified base sequence can be OR10G4, which corresponds to published data (Mainland JD et al., Nature Neuroscience 17, 114-120 (2014)).

It should be noted that when fluorescence intensity based on changes in the concentration of intracellular Ca²⁺ is repeatedly quantified on different cells in the method of 5), it is expected that the CV value is around 5%, which is extremely stable.

As the same results, published OR5P3 (coumarin responsive human-derived olfactory receptor: Saito H et al., Science Signaling 2, ra9 (2009)) and OR8D1 (caramel furanone responsive human-derived olfactory receptor: Mainland JD et al., Nature Neuroscience 17, 114-120 (2014)) are identified by other odor molecules, 1 mM coumarin and 1 mM caramel furanone, respectively, and thus it is expected that a human-derived olfactory receptor corresponding to an optional odor molecule can be isolated by using the method.

In addition, when F293-C1/2 strain is transformed using 404 types of human-derived olfactory receptor-expressing plasmid produced in the above 6) and pOF44 plasmid (Invitrogen) in the same manner, hygromycin-resistant and zeocin-sensitive cells are selected, and the same assay as above is carried out, it is expected that OR10G4 is isolated as a human-derived olfactory receptor which responds to vanillin.

### 11) Method for making a human olfactory receptor-expressing cell array

Of the 404 types of human-derived olfactory receptor-expressing plasmid produced in the above 6), 1 mL (DNA amount: 500 ng) of pcDNA5/FRT-Rhod-KP290534.1 is mixed with 1 mL of Lipofectamine 2000 (Invitrogen), a gene transfection reagent, and this is dot-printed in 100 pL each in the form of array on the substrate. In the substrate used, hydrophilicity is increased by plasma treatment of the surface of a slide glass (Matsunami Glass Ind., Ltd.) by a plasma modifier (PM100, Yamato Scientific Co., Ltd.) It should be noted that a machine used for dot printing is a LaboJet-500 manufactured by MicroJet. Here, a complex including a plasmid to be immobilized on the substrate and a gene transfection reagent is arranged so that the diameter is about 50 mm.

### 12) Isolation of a human olfactory receptor which responds to an arbitrary odor using a human olfactory receptor-expressing cell array

The above human-derived olfactory receptor-expressing plasmid is immobilized on each of the 404 sites of a slide glass for array in the method of the above 11), and a HEK293-C1/2 strain is added in an equal amount from the upper part of each of the immobilized sites. When the array is left to stand on specific culture conditions and observed after 2 days, it is expected that added HEK293-C1/2 strain is proliferated on the sites on which the plasmid has been immobilized to form colonies.

When a slide glass for array on which an EGFP (green fluorescent proteins)-expressing plasmid is immobilized in the same manner is investigated as a control at the same time, it is expected that colonies are formed on all the immobilized sites and the expression of EGFP can be observed.

After that, the substrate having formed colonies thereon is loaded on the above OneCell system in the method shown in 2). When this was stimulated using an optional odor molecule, vanillin, in a concentration of 10 mM in the method shown in 3), an increase in fluorescence intensity was observed in some cells.

It is expected that a HEK293-C1/2 strain grown in the form of a colony exists on the sites on which the above-described OR10G4-expressing plasmid has been immobilized. It should be noted that when fluorescence intensity based on changes in the concentration of intracellular Ca²⁺ is repeatedly quantified on different cells in the method shown in 5), it is expected that the CV value is around 5%, which is extremely stable.

When this procedure is carried out using 1 mM coumarin and 1 mM caramel furanone in the same manner, it is expected that a HEK293-C1/2 strain grown in the form of a colony exists on sites on which the above-described OR5P3 and OR8D1-expressing plasmids respectively are immobilized.

From the above, it is expected that a human-derived olfactory receptor corresponding to an arbitrary odor molecule can be isolated using this method without cell collection.

### 13) Making a human olfactory receptor-expressing cell array and measuring activation degree by calculation method (B)

An example in which a method for making a human olfactory receptor-expressing cell array in the above 11) and 12) is applied to the array 1 and receptor array 10r of the present invention will now be described. In the substrate 5 used, hydrophilicity is increased by plasma treatment of the slide glass (Matsunami Glass Ind., Ltd.) In addition, the hydrophobic coating film 6 is a fluorine resin and the hydrophobic coating film 6 having a plurality of through-holes 2 is formed on the substrate 5 by screen printing. Here, the contact angle of the hydrophobic coating film 6 using water at 23°C was 150°.

Of the 404 types of human-derived olfactory receptor-expressing plasmid produced in the above 6), 1 mL (DNA amount: 500 ng) of pcDNA5/FRT-Rhod-KP290534.1 was mixed with 1 mL of Lipofectamine 2000 (Invitrogen), a gene transfection reagent, and this was dot-printed in 100 pL each on each through-hole 2. Here, a complex including a plasmid to be immobilized on the substrate 5 and a gene transfection reagent is printed so that the diameter is about 50 mm. An array 1 was completed as described above.

Subsequently, a HEK293-C1/2 strain was added to each through-hole 2 of the array 1 in an equal amount, and a plurality of eukaryotic cells were held in one through-hole 2 to complete a eukaryotic cell array 10.

Subsequently, incubation was carried out for 2 days on specific culture conditions. A HEK293-C1/2 strain was proliferated, and transient expression was induced in a plurality of cells in the interior of each through-hole 2 to form a plurality of receptors. As described above, a receptor array 10r was completed.

A latex • ringer solution was brought into contact with the receptor array 10r as a test substance, and an activation degree was measured by the calculation method (B). Here, the latex • ringer solution is a solution obtained by heating latex at 250°C and collecting generated gas in ringer solution. When measuring the activation degree, a Calbryte 520AM solution, a calcium ion-measuring fluorescent indicator, was used, and changes in the concentration of intracellular calcium ion were observed as fluorescence intensity. Here, the Calbryte 520AM solution is a solution which includes Calbryte 520AM and Pluronic F-127, a surface activating agent, and has ringer solution as a solvent. In addition, the first prescribed time for the measurement of fluorescence intensity is for 5 minutes from 6 minutes to 1 minute before the contact with a test substance (T0 to T300 seconds), and the second prescribed time is for 10 minutes from 0 minutes to 10 minutes after the contact with a test substance (T360 to T900 seconds).

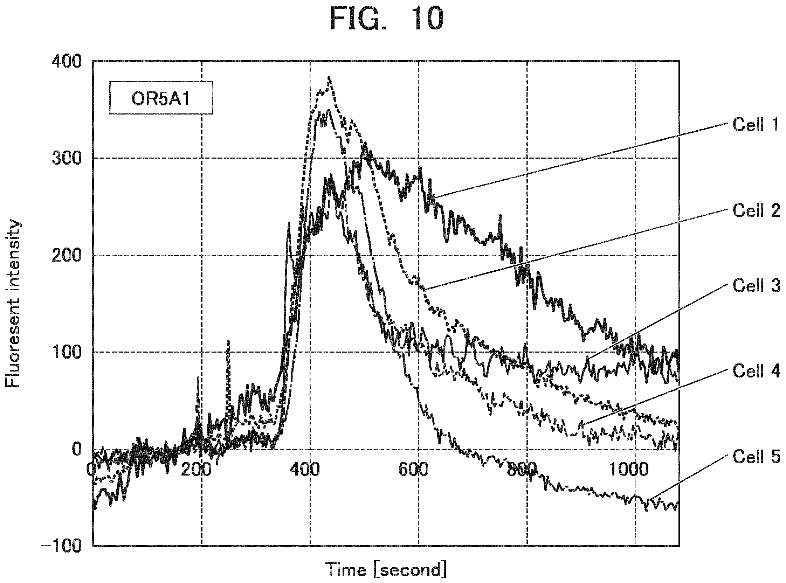

As for a receptor OR5A1, measured fluorescence intensity of 5 eukaryotic cells expressing the receptor OR5A1 is shown in Fig. 10. In Fig. 10, time (sec) is taken along the abscissa and fluorescence intensity is taken along the ordinate. Five curves each show 5 cells (Cell 1 to Cell 5). It is found that the cells each have different response properties to a test substance; however, the tendency is very similar (note that an origin correction is carried out using Fbase as 0 in Fig. 10). Each activation degree (Fmax/Fbase) of these 5 cells was calculated, and 5 average values, standard deviations and coefficients of variation were found. The average value was 1.5, the standard deviation was 0.08 and the CV value (coefficient of variation) was 5%, which verified that measurement could be carried out with very high reproducibility.

As for 7 different receptors, the activation degree of 4 or more cells in each receptor was measured by the same means and the data were investigated. The results are shown in Table 1 (Table 1 also includes the measurement values of the receptor OR5A1). All the receptors had a CV value (coefficient of variation) of 7% or less, which verified that measurement could be carried out with high reproducibility.

**[Table 1]**

| Receptor name | Number of effective cells | Activation degree | | CV value (%) |
|---|---|---|---|---|
| | | Average | Standard deviation | |
| OR1S1 | 4 | 1.4 | 0.06 | 4 |
| OR10G8 | 4 | 1.4 | 0.08 | 6 |
| OR5D18 | 5 | 1.5 | 0.04 | 2 |
| OR5W2 | 5 | 1.4 | 0.06 | 4 |
| OR8K1 | 5 | 1.4 | 0.08 | 6 |
| OR5A1 | 5 | 1.5 | 0.08 | 5 |
| OR8K5 | 6 | 1.5 | 0.06 | 4 |
| OR4C15 | 7 | 1.5 | 0.10 | 7 |

### 14) Image data processing

The method for measuring an activation degree of the present invention includes a step of collectively acquiring a region including all through-holes 2 as image data at the same time, individually recognizing eukaryotic cells in the through-holes 2, and obtaining each fluorescence of each eukaryotic cell as a numerical value. Such individual recognition, and individual data processing and arithmetic processing of each single cell can be carried out using a known image processing software.

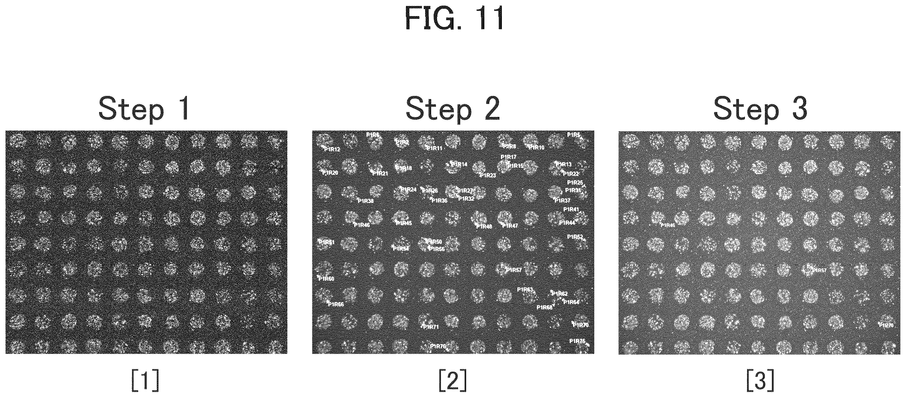

Figs. 11 show a case where image data of a receptor array 10r is collectively acquired and individual recognition on a cell basis is carried out.

Fig. 11[1] is a difference image between an image 1 minute before the contact with a test substance and an image 1 minute after the contact with the test substance as an example of image processing (Step 1). Ninety nine through-holes 2 provided in the hydrophobic coating film 6 can be collectively visible. It is found that a plurality of cells are held in each through-hole 2.

Fig. 11[2] is an image obtained by recognizing cells with a fluorescence intensity of 200 (slice level) or more in the image data in Step 1 on a cell basis and labeling them on a cell basis as an example of individual data processing. In this image sample, it is collectively visible that up to two cells show a fluorescence intensity of 200 or more in a through-hole 2.

Fig. 11[3] is an image obtained by carrying out an operation on a cell basis from images before and after the contact with a test substance, judging cells having Fmax ≥ Fbase + 5 SD Step on a cell basis, and labeling them on a cell basis as an example of arithmetic processing. In this image sample, three cells are used in the whole region by judgement.

As described above, even when a plurality of eukaryotic cells are held in a through-hole 2, cells can be individually recognized one by one, and prescribed arithmetic processing can be sufficiently carried out on a cell basis.

### 15) Formation of odor (1)

Melon odor is used as a target substance, and the target substance is formed from reference substances (cucumber odor, banana odor, mayonnaise odor).

### Sample preparation

Samples (melon, cucumber, banana, mayonnaise) of 3 cubic centimeters each were put on the bottom of a 30 mL vial, and a silica monolith trap agent (Mono Trap RGPS TD (manufactured by GL Sciences Inc.)) was put on the upper portion thereof without contact with the sample. After 24 hours at room temperature, the silica monolith trap agent was taken out, and quickly transferred to a portable thermal desorber Handy TD TD265 (manufactured by GL Sciences Inc.) The temperature was raised to 350°C at a gradient of 30°C for 1 second, and an odor component discharged into ringer solution was trapped by bubbling. The composition of a ringer solution is as follows, and a pH 7.2 mixed solution is stored at 4°C:
NaCl 140 mM
KCl 5 mM
CaCl₂ 1 mM
MgCl₂ 1 mM
HEPES 10 mM
D-Glucose 10 mM
Sodium pyruvate 1 mM

The obtained ringer solution including an odor component derived from a sample was directly used for an exhaustive odor analysis using a human olfactory receptor-expressing array (receptor array 10r), that is, the ringer solution including an odor component was used as a test substance, and the activation degree of each receptor was measured. A measurement method is the same as the measurement of activation degree described in 13).

### Analysis:

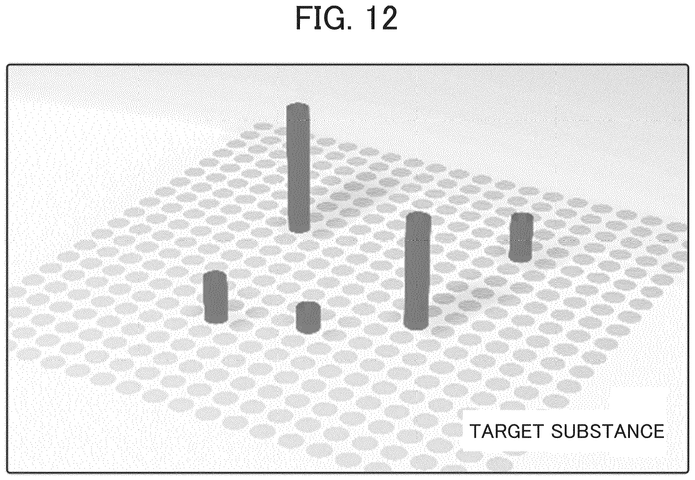

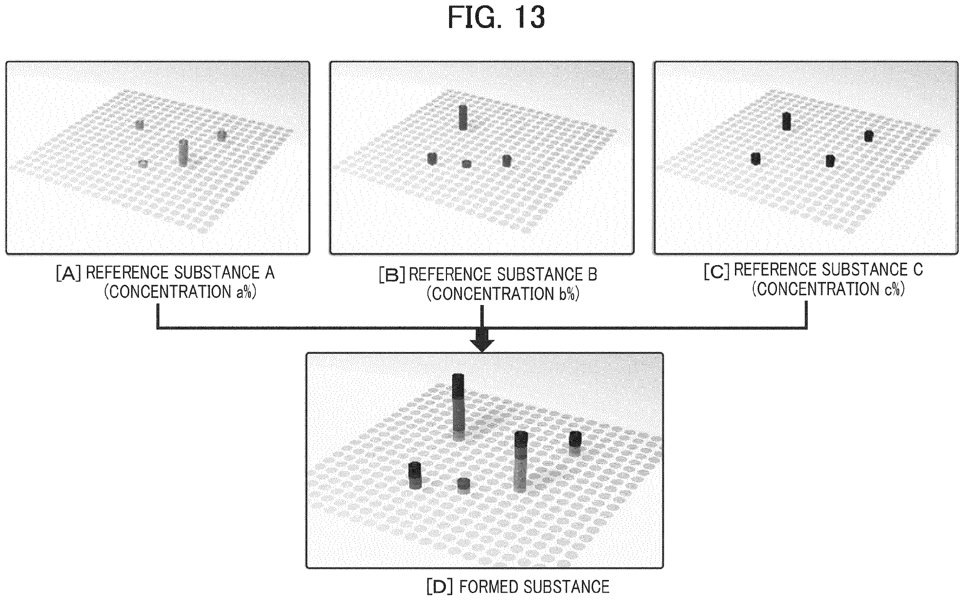

The degree of activation of human olfactory receptors by an odor derived from each sample was visualized by an odor matrix. The odor matrix is a concept that each lattice point of the matrix corresponds to each human olfactory receptor. Specifically, a total of 400 types, including human olfactory receptors (396 types), two types of negative control receptor (expressing GPCR, which is not an olfactory receptor), and two types of cells which have not expressed a receptor, are expressed by 20 x 20 lattice points. The melon odor, a target substance, is shown in Fig. 12. In addition, the cucumber odor, a reference substance A, is shown in Fig. 13[A], the banana odor, a reference substance B, in Fig. 13[B], and the mayonnaise odor, a reference substance C, in Fig. 13[C]. From the data of these odor matrices, in order to form melon, the target substance, the banana odor and mayonnaise odor may be mixed using the cucumber odor as a base, and specifically, it was judged that mixing a reference substance A (concentration a%) a = 70%, a reference substance B (concentration b%) b = 20%, and a reference substance C (concentration c%) c = 10% was optimal. An odor matrix computationally formed by the percentages is shown in Fig. 13[D]. The target substance (Fig. 12) and a computationally formed substance (Fig. 13[D]) have similar forms to each other. Next, the ringer solutions each including an odor component were mixed by the above mixing ratio to obtain a formed melon odor.

### Sensory test:

A sensory test of the above formed melon odor was carried out in accordance with QDA method (quantitative descriptive sensory analysis) described in IMAMURA Miho "KAGAKU TO SEIBUTSU" (Vol. 50, No. 11, 2012). The used samples were 1 mL of ringer solutions, each including the formed melon odor made in the above method, the cucumber odor, banana odor, mayonnaise odor or melon odor, and moreover 1 mL of ringer solutions, each including the watermelon odor, pumpkin odor, peach odor or melon soda odor were prepared as similar odors. When 20 voluntary sensory testers evaluated the above samples (9 types in total), 18 of 20 testers judged that the formed melon odor and the melon odor (original melon odor), the target substance, had the most characteristic notes in common. On the other hand, no one judged that the formed melon odor and samples other than the melon odor (which was the target substance), had the most characteristic notes in common.

### 16) Formation of odor (2)

A ringer solution including an odor component derived from milk, a ringer solution including an odor component derived from yellow pickled radish, and a ringer solution including an odor component derived from corn soup were prepared in the same method as in 15). In order to construct the odor matrix of the corn soup odor, it was judged that mixing equal amounts of a ringer solution including an odor component derived from milk and a ringer solution including an odor component derived from yellow pickled radish were suitably from the odor matrix of the milk odor and the odor matrix of the yellow pickled radish odor was suitable. When the formed corn soup odor thus obtained, the corn soup odor (original corn soup odor) which was a target substance, and moreover a fresh cream odor, a clam chowder odor and a consommé odor produced in the same manner were evaluated in the same sensory test as in 15), 17 of 20 voluntary sensory testers judged that the formed corn soup odor and the corn soup odor (original corn soup odor) which was a target substance, had the most characteristic notes in common. On the other hand, no one judged that the formed corn soup odor and samples other than the corn soup odor which was the target substance, had the most characteristic notes in common.

### EXPLANATION OF REFERENCE NUMERALS

- 1:: Array
- 2:: Through-hole
- 3:: Nucleic acid
- 4:: Eukaryotic cell region
- 4A1:: Eukaryotic cell
- 5:: Substrate
- 6:: Hydrophobic coating film
- 10:: Eukaryotic cell array

## Claims

1. An array, comprising
a substrate, and a hydrophobic coating film on the substrate,
wherein the hydrophobic coating film has a plurality of through-holes, and
a nucleic acid comprising a gene which codes for a prescribed receptor is provided in contact with the substrate in the interior of the through-holes.

2. The array according to claim 1, wherein nucleic acids comprising genes which code for the described receptors which are different from each other are provided in each of the through-holes.

3. The array according to claim 1 or 2, wherein an average cross-sectional area value of the plurality of through-holes is 0.125 mm² or more and 0.283 mm² or less.

4. The array according to claim 3, wherein 200 or more of the through-holes are provided in the substrate and an average pitch between the through-holes is 0.6 mm or more and 0.8 mm or less.

5. The array according to claims 1 to 4, wherein the hydrophobic coating film has a contact angle with water at 23°C of 70° or more.

6. The array according to claims 1 to 5, wherein the prescribed receptor comprises a G protein-coupled receptor.

7. The array according to claim 6, wherein the G protein-coupled receptor comprises an olfactory receptor.

8. The array according to claims 1 to 7, wherein a plurality of eukaryotic cells are further held in one of the through-holes.

9. Eukaryotic cells, which express CNGA2 and GNAL or mutants thereof as foreign genes.

10. The cells according to claim 9, which further express CNGA4 and/or CNGBlb or mutants thereof as foreign genes.

11. The cells according to claim 9 or 10, which further express a gene which codes for a protein which can transfer an olfactory receptor expressed in the cells from an endoplasmic reticulum membrane to a cell membrane to raise an efficiency of presentation of the olfactory receptor to a surface of the cells.

12. At least one vector, which is selected from a group consisting of:
(1) Vector A having genes which code for CNGA2 and GNAL or mutants thereof,
(2) Vector B having genes which code for CNGA4 and/or CNGBlb or mutants thereof, and
(3) Vector C having a gene which codes for a protein which can transfer an olfactory receptor expressed in cells from an endoplasmic reticulum membrane to a cell membrane to raise an efficiency of presentation of the olfactory receptor to a surface of the cells.

13. Eukaryotic cells, into which at least one vector according to claim 12 is incorporated.

14. Eukaryotic cells having a region to incorporate a certain number of genes which code for olfactory receptors derived from a mammal into a chromosome of cells according to any of claims 9 to 11 or 13.

15. Eukaryotic cells, in which a human-derived olfactory receptor gene is incorporated into the region according to claim 14.

16. The array according to claim 8, wherein the eukaryotic cells are eukaryotic cells according to claims 9 to 11 or 13 to 15.

17. A method for calculating an activation degree of a prescribed receptor by a test substance, the method comprising:
(1) a step of measuring, when the test substance is brought into contact with a plurality of eukaryotic cells expressing the receptor, an amount of ions taken into each of the cells,
(2) a step of measuring, when the same cells as used in measurement in step 1 are depolarized, an amount of ions in each of the cells, and
(3) a step of calculating a ratio of a numerical value measured in step 1 and a numerical value measured in step 2.

18. The method according to claim 17,
wherein, in step 1 and step 2,
a means for measuring the amount of ions taken into the cells is a means using an ion-measuring pigment or a means using an ion-binding fluorescent protein.

19. The method according to claim 17 or 18, wherein the depolarization step according to step 2 is a step of applying a potassium compound or ionophore to the eukaryotic cells.

20. A method for calculating an activation degree of a receptor by a test substance,
the method comprising:
a step of measuring, when the test substance is brought into contact with a plurality of eukaryotic cells expressing the receptor, an amount of a substance taken into each of the cells,
wherein, when an average value of fluorescence in a first prescribed time before the contact is Fbase and
a greatest value of fluorescence in a second prescribed time after the contact is Fmax,
Fmax/Fbase is calculated and used as an activation degree.

21. The method according to claim 20, wherein, when a standard deviation of fluorescence in the first prescribed time is used as SD and
the Fmax is not less than Fbase + 5 SD, the Fmax is used as an activation degree, and
when the Fmax is less than Fbase + 5 SD, the Fmax is not used as an activation degree.

22. The method according to claim 20 or 21, wherein after the measurement step in regard to a certain test substance a positive control substance is not brought into contact with the eukaryotic cells and a next test substance is brought into contact with eukaryotic cells.

23. The method according to claim 22, wherein contact with the next test substance is carried out at a time when a fluorescence value by the certain test substance is not less than Fbase + 5 SD.

24. The method according to claims 17 to 23, wherein a receptor expression of the eukaryotic cells is a transient expression.

25. The method according to claim 24, wherein the receptor is an olfactory receptor.

26. A method for measuring an activation degree of a receptor by a test substance,
the method comprising:
a step of allowing an ion-measuring pigment or an ion-binding fluorescent protein to act on the array according to claim 16,
a step of bringing the test substance into contact with the array,
a step of simultaneously acquiring a region comprising all the through-holes as image data,
a step of individually recognizing the eukaryotic cells and obtaining fluorescence in each of the eukaryotic cells as a numerical value, and
a step of calculating an activation degree by arithmetic processing of the numerical value.

27. The method according to claim 26,
wherein the arithmetic processing is processing by which, when an average value of fluorescence in a prescribed first time before the contact is Fbase and
a greatest value of fluorescence in a second prescribed time after the contact is Fmax,
Fmax/Fbase is calculated and used as an activation degree.

28. The method according to claim 27,
wherein, when a standard deviation of fluorescence in a prescribed first time before the contact is SD and
the Fmax is not less than Fbase + 5 SD, the arithmetic processing uses the Fmax as an activation degree, and,
when the Fmax is less than Fbase + 5 SD, the arithmetic processing does not use the Fmax as an activation degree.

29. The method according to claims 26 to 28, wherein the receptor is a human-derived olfactory receptor.

30. A method for forming an odor of a target substance,
wherein two or more reference substances are combined so that based on an activation degree of each receptor by the two or more reference substances measured using the method according to claim 29,
an activation degree of each receptor by the target substance measured using the measurement method is formed.

31. A method for producing a composition having a formed odor of a target substance,
wherein two or more reference substances are combined so that based on an activation degree of each receptor by the two or more reference substances measured using the method according to claim 29,
an activation degree of each receptor by the target substance measured using the measurement method is formed.

32. The method according to claim 30 or 31, wherein the combination method is an operation of a first data and a second data,
wherein an activation degree of each receptor by each of the reference substances is obtained as the first data, and
an activation degree of each receptor by the target substance is obtained as the second data.

33. A method for screening a substance to modify a sample odor into a target odor,
the method comprising a step of selecting a candidate substance as an indicator that an activation degree of each receptor by the sample measured using the measurement method according to claim 29 is brought close to an activation degree of each receptor in the target odor state measured using the measurement method according to claim 29.

34. The method according to claim 33, wherein selection is carried out based on whether or not an activation degree of each of the receptors measured when adding a candidate substance to the sample is brought close to the activation degree in the target odor state.

35. The method according to claim 33, wherein the selection comprises selection from reference substances based on existing information about an activation degree of each of the receptors by the reference substances.


SEQUENCE LISTING

<110>  Komi Hakko Corporation

<120>  Method for quantifying odor, cell used for thereof, and method
       for producing thereof

<130>  ATF-317PCT

<150>  JP2017-157492

<151>  2017-08-17

<160>  12

<170>  PatentIn version 3.5

<210>  1
<211>  1995
<212>  DNA
<213>  Homo sapiens

<400>  1
atgaccgaaa aaaccaatgg tgtgaagagc tccccagcca ataatcacaa ccatcatgca      60

cctcctgcca tcaaggccaa tggcaaagat gaccacagga caagcagcag gccacactct     120

gcagctgacg atgacacctc ctcagaactg cagaggctgg cagacgtgga tgccccacag     180

cagggaagga gtggcttccg caggatagtt cgcctggtgg ggatcatcag agaatgggcc     240

aacaagaatt tccgagagga ggaacctagg cctgactcat tcctcgagcg ttttcgtggg     300

cctgaactcc agactgtgac cacacaggag ggggatggca aaggcgacaa ggatggcgag     360

gacaaaggca ccaagaagaa atttgaacta tttgtcttgg acccagctgg ggattggtac     420

tactgctggc tatttgtcat tgccatgccc gtcctttaca actggtgcct gctggtggcc     480

agagcctgct tcagtgacct acagaaaggc tactacctgg tgtggctggt gctggattat     540

gtctcagatg tggtctacat tgcggacctc ttcatccgat tgcgcacagg tttcctggag     600

caggggctgc tggtcaaaga taccaagaaa ctgcgagaca actacatcca caccctgcag     660

ttcaagctgg atgtggcttc catcatcccc actgacctga tctattttgc tgtggacatc     720

cacagccctg aggtgcgctt caaccgcctg ctgcactttg cccgcatgtt tgagttcttt     780

gaccggacag agacacgcac caactaccct aacatcttcc gcatcagcaa ccttgtcctc     840

tacatcttgg tcatcatcca ctggaatgcc tgcatctatt atgccatctc caaatccata     900

ggctttgggg tcgacacctg ggtttaccca aacatcactg accctgagta tggctacctg     960

gctagggaat acatctattg cctttactgg tccacactga ctctcactac cattggggag    1020

acaccacccc ctgtaaagga tgaggagtac ctatttgtca tctttgactt cctgattggc    1080

gtcctcatct ttgccaccat cgtgggaaat gtgggctcca tgatctccaa catgaatgcc    1140

acccgggcag agttccaggc taagatcgat gccgtgaaac actacatgca gttccgaaag    1200

gtcagcaagg ggatggaagc caaggtcatt aggtggtttg actacttgtg gaccaataag 1260

aagacagtgg atgagcgaga aattctcaag aatctgccag ccaagctcag ggctgagata 1320

gccatcaatg tccacttgtc cacactcaag aaagtgcgca tcttccatga ttgtgaggct 1380

ggcctgctgg tagagctggt actgaaactc cgtcctcagg tcttcagtcc tggggattac 1440

atttgccgca aaggggacat cggcaaggag atgtacatca ttaaggaggg caaactggca 1500

gtggtggctg atgatggtgt gactcagtat gctctgctgt cggctggaag ctgctttggc 1560

gagatcagta tccttaacat taagggcagt aaaatgggca atcgacgcac agctaatatc 1620

cgcagcctgg gctactcaga tctcttctgc ttgtccaagg atgatcttat ggaagctgtg 1680

actgagtacc ctgatgccaa gaaagtccta gaagagaggg gtcgggagat cctcatgaag 1740

gagggactgc tggatgagaa cgaagtggca accagcatgg aggtcgacgt gcaggagaag 1800

ctagggcagc tggagaccaa catggaaacc ttgtacactc gctttggccg cctgctggct 1860

gagtacacgg gggcccagca gaagctcaag cagcgcatca cagttctgga aaccaagatg 1920

aaacagaaca atgaagatga ctacctgtct gatgggatga acagccctga gctggctgct 1980

gctgacgagc cataa 1995

<210> 2
<211> 1146
<212> DNA
<213> Homo sapiens

<400> 2
atggggtgtt tgggcggcaa cagcaagacg acggaagacc agggcgtcga tgaaaaagaa 60

cgacgcgagg ccaacaaaaa gatcgagaag cagttgcaga aagagcgcct ggcttacaag 120

gctacccacc gcctgctgct cctgggggct ggtgagtctg ggaaaagcac tatcgtcaaa 180

cagatgagga tcctgcacgt caatgggttt aatcccgagg aaaagaaaca gaaaattctg 240

gacatccgga aaaatgttaa agatgctatc gtgacaattg tttcagcaat gagtactata 300

atacctccag ttccgctggc caaccctgaa aaccaatttc gatcagacta catcaagagc 360

atagccccta tcactgactt tgaatattcc caggaattct ttgaccatgt gaaaaaactt 420

tgggacgatg aaggcgtgaa ggcatgcttt gagagatcca acgaatacca gctgattgac 480

tgtgcacaat acttcctgga aagaatcgac agcgtcagct tggttgacta cacacccaca 540

gaccaggacc tcctcagatg cagagttctg acatctggga tttttgagac acgattccaa 600

gtggacaaag taaacttcca catgtttgat gttggtggcc agagggatga gaggagaaaa 660

tggatccagt gctttaacga tgtcacagct atcatttacg tcgcagcctg cagtagctac 720

aacatggtga ttcgagaaga taacaacacc aacaggctga gagagtccct ggatcttttt 780

gaaagcatct ggaacaacag gtggttacgg accatttcta tcatcttgtt cttgaacaaa 840

caagatatgc tggcagaaaa agtcttggca gggaaatcaa aaattgaaga ctatttccca 900

gaatatgcaa attatactgt tcctgaagac gcaacaccag atgcaggaga agatcccaaa 960

gttacaagag ccaagttctt tatccgggac ctgtttttga ggatcagcac ggccaccggt 1020

gacggcaaac attactgcta cccgcacttc acctgcgccg tggacacaga gaacatccgc 1080

agggtgttca acgactgccg cgacatcatc cagcggatgc acctcaagca gtatgagctc 1140

ttgtga 1146

<210> 3
<211> 1728
<212> DNA
<213> Homo sapiens

<400> 3
atgagccagg acaccaaagt gaagacaaca gagtccagtc ccccagcccc atccaaggcc 60

aggaagttgc tgcctgtcct ggacccatct ggggattact actactggtg gctgaacaca 120

atggtcttcc cagtcatgta taacctcatc atcctcgtgt gcagagcctg cttccccgac 180

ttgcagcacg gttatctggt ggcctggttg gtgctggact acacgagtga cctgctatac 240

ctactagaca tggtggtgcg cttccacaca ggattcttgg aacagggcat cctggtggtg 300

gacaagggta ggatctcgag tcgctacgtt cgcacctgga gtttcttctt ggacctggct 360

tccctgatgc ccacagatgt ggtctacgtg cggctgggcc cgcacacacc caccctgagg 420

ctgaaccgct ttctccgcgc gccccgcctc ttcgaggcct tcgaccgcac agagacccgc 480

acagcttacc caaatgcctt tcgcattgcc aagctgatgc tttacatttt tgtcgtcatc 540

cattggaaca gctgcctata ctttgcccta tcccggtacc tgggcttcgg gcgtgacgca 600

tgggtgtacc cggaccccgc gcagcctggc tttgagcgcc tgcggcgcca gtacctctat 660

agcttttact tctccacgct gatactgact acagtgggcg atacaccgcc gccagccagg 720

gaagaagagt acctcttcat ggtgggcgac ttcctgctgg ccgtcatggg tttcgccacc 780

atcatgggta gcatgagctc tgtcatctac aacatgaaca ctgcagatgc ggctttctac 840

ccagatcatg cactggtgaa gaagtacatg aagctgcagc acgtcaaccg caagctggag 900

cggcgagtta ttgactggta tcagcacctg cagatcaaca agaagatgac caacgaggta 960

gccatcttac agcacttgcc tgagcggctg cgggcagaag tggctgtgtc tgtgcacctg 1020

tccactctga gccgggtgca gatctttcag aactgtgagg ccagcctgct ggaggagctg 1080

gtgctgaagc tgcagcccca gacctactca ccaggtgaat atgtatgccg caaaggagac 1140

attggccaag agatgtacat catccgagag ggtcaactgg ccgtggtggc agatgatggt 1200

atcacacagt atgctgtgct cggtgcaggg ctctactttg gggagatcag catcatcaac 1260

atcaaaggga acatgtctgg gaaccgccgc acagccaaca tcaagagcct aggttattca 1320

gacctattct gcctgagcaa ggaggacctg cgggaggtgc tgagcgagta tccacaagca 1380

cagaccatca tggaggagaa aggacgtgag atcctgctga aaatgaacaa gttggacgtg 1440

aatgctgagg cagctgagat cgccctgcag gaggccacag agtcccggct acgaggccta 1500

gaccagcagc tggatgatct acagaccaag tttgctcgcc tcctggctga gctggagtcc 1560

agcgcactta agattgctta ccgcattgaa cggctggagt ggcagactcg agagtggcca 1620

atgcccgagg acctggctga ggctgatgac gagggtgagc ctgaggaggg aacttccaaa 1680

gatgaagagg gcagggccag ccaggaggga cccccaggtc cagagtga 1728

<210> 4
<211> 2607
<212> DNA
<213> Homo sapiens

<400> 4
atggctacac catgtcagaa catggagctg aattgcctag tccaggacca gatacctggc 60

caggtggcct ctgcctctgc cctccaaaac ctggtagagc atctccctaa tgtgcccagc 120

taccgcatcc caatcacccg catccctgtc ctcacctccc ggagaaccag cttgtccaac 180

tccagcttcg ccaaggagac caggagctcc atccgccaac tagtgcctgc cacgaaacag 240

cacccagaag tgcaggtgga agatactgat gctgatagct gccccctcat ggcagaagag 300

aatccaccct caaccgtgtt gccgccaccg tctcctgcca aatcagacac ccttatagtc 360

ccaagctcag cctcggggac acacaggaag aagctgccct ctgaggatga tgaggctgaa 420

gagctcaagg cgttgtcacc agcagagtcc ccagtggttg cctggtctga ccccaccacc 480

ccgaaggaca ctgatggcca ggaccgtgcg gcctccacgg ccagcacaaa tagcgccatc 540

atcaacgacc ggctccagga gctggtgaag ctcttcaagg agcggacaga gaaagtgaag 600

gagaaactca ttgaccctga cgtcacctct gatgaggaga gccccaagcc ctccccagcc 660

aagaaagccc cagagccagc tccagacaca aagcccgctg aagccgagcc agtggaagag 720

gagcactatt gcgacatgct ctgctgcaag ttcaaacacc gcccctggaa gaagtaccag 780

tttccccaga gcattgaccc gctgaccaac ctgatgtatg tcctatggct gttcttcgtg 840

gtgatggcct ggaattggaa ctgttggctg attcccgtgc gctgggcctt cccctaccag 900

accccggaca acatccacca ctggctgctg atggattacc tatgcgacct catctacttc 960

ctggacatca ccgtgttcca gacacgcctg cagtttgtca gaggcgggga catcattacg 1020

gacaaaaagg acatgcgaaa taactacctg aagtctcgcc gcttcaagat ggacctgctc 1080

agcctcctgc ccttggattt tctctatttg aaagtcggtg tgaaccccct cctccgcctg 1140

ccccgctgtt taaagtacat ggccttcttc gagtttaaca gccgcctgga atccatcctc 1200

agcaaagcct acgtgtacag ggtcatcagg accacagcct accttctcta cagcctgcat 1260

ttgaattcct gtctttatta ctgggcatcg gcctatcagg gcctcggctc cactcactgg 1320

gtttacgatg gcgtgggaaa cagttatatt cgctgttact actttgctgt gaagaccctc 1380

atcaccatcg gggggctgcc tgaccccaag acactctttg aaattgtctt ccagctgctg 1440

aattatttca cgggcgtctt tgctttctct gtgatgatcg gacagatgag agatgtggta 1500

ggggccgcca ccgccggaca gacctactac cgcagctgca tggacagcac ggtgaagtac 1560

atgaatttct acaagatccc caagtccgtg cagaaccgcg tcaagacctg gtacgagtac 1620

acctggcact cgcaaggcat gctggatgag tcagagctga tggtgcagct tccagacaag 1680

atgcggctgg acctcgccat cgacgtgaac tacaacatcg ttagcaaagt cgcactcttt 1740

cagggctgtg accggcagat gatctttgac atgctgaaga ggcttcgctc tgttgtctac 1800

ctgcccaacg actatgtgtg caagaagggg gagatcggcc gtgagatgta catcatccag 1860

gcagggcaag tgcaggtctt gggcggccct gatgggaaat ctgtgctggt gacgctgaaa 1920

gctggatctg tgtttggaga aataagcttg ctggctgttg ggggcgggaa ccggcgcacg 1980

gccaacgtgg tggcgcacgg gtttaccaac ctcttcatcc tggataagaa ggacctgaat 2040

gagattttgg tgcattatcc tgagtctcag aagttactcc ggaagaaagc caggcgcatg 2100

ctgagaagca acaataagcc caaggaggag aagagcgtgc tgatccttcc accccgggcg 2160

ggcaccccaa agctcttcaa cgctgccctc gctatgacag gaaagatggg tggcaagggg 2220

gcaaaaggcg gcaaacttgc tcacctccgg gcccggctca aagaactggc cgcgctggag 2280

gcggctgcaa agcagcaaga gttggtggaa caggccaaga gctcgcaaga cgtcaaggga 2340

gaggaaggct ccgccgcccc agaccagcac acgcacccaa aggaggccgc caccgaccca 2400

cccgcgcccc ggacgccccc cgagcccccg gggtctccac cgagctctcc accgcctgcc 2460

tcccttggga ggccggaggg agaggaggag gggccggccg agcccgaaga gcactcggtg 2520

aggatctgca tgagcccggg cccggagccg ggagagcaga tcctgtcggt gaagatgccg 2580

gaggaaaggg aggagaaggc ggagtaa 2607

<210> 5
<211> 792
<212> DNA
<213> Homo sapiens

<400> 5
atgaggattt ttagaccgtg gagactgcgc tgccctgccc tgcacctacc ctcactctcc 60

gtgttctcac taaggtggaa attgccttcc ctcactactg acgagaccat gtgtaaaagc 120

gtgaccacag atgagtggaa gaaagtcttc tatgagaaga tggaggaggc aaagccggct 180

gacagctggg acctcatcat agaccccaac ctcaagcaca atgtgctgag ccctggttgg 240

aagcagtacc tggaattgca tgcttcaggc aggttccact gctcctggtg ctggcacacc 300

tggcagtcgc cctacgtggt catcctcttc cacatgttcc tggaccgcgc ccagcgggcg 360

ggctcggtgc gcatgcgcgt cttcaagcag ctgtgctatg agtgcggcac ggcgcggctg 420

gacgagtcca gcatgctgga ggagaacatc gagggcctgg tggacaacct catcaccagc 480

ctgcgcgagc agtgctacgg cgagcgtggc ggccagtacc gcatccacgt ggccagccgc 540

caggacaacc ggcggcaccg cggagagttc tgcgaggcct gccaggaggg catcgtgcac 600

tggaagccca gcgagaagct gctggaggag gaggcgacca cctacacctt ctcccgggcg 660

cccagcccca ccaagtcgca ggaccagacg ggctcaggct ggaacttctg ctctatcccc 720

tggtgcttgt tttgggccac ggtcctgctg ctgatcatct acctgcagtt ctctttccgt 780

agctccgtat aa 792

<210> 6
<211> 684
<212> DNA
<213> Homo sapiens

<400> 6
atgtgtaaaa gcgtgaccac agatgagtgg aagaaagtct tctatgagaa gatggaggag 60

gcaaagccgg ctgacagctg ggacctcatc atagacccca acctcaagca caatgtgctg 120

agccctggtt ggaagcagta cctggaattg catgcttcag gcaggttcca ctgctcctgg 180

tgctggcaca cctggcagtc gccctacgtg gtcatcctct tccacatgtt cctggaccgc 240

gcccagcggg cgggctcggt gcgcatgcgc gtcttcaagc agctgtgcta tgagtgcggc 300

acggcgcggc tggacgagtc cagcatgctg gaggagaaca tcgagggcct ggtggacaac 360

ctcatcacca gcctgcgcga gcagtgctac ggcgagcgtg gcggccagta ccgcatccac 420

gtggccagcc gccaggacaa ccggcggcac cgcggagagt tctgcgaggc ctgccaggag 480

ggcatcgtgc actggaagcc cagcgagaag ctgctggagg aggaggcgac cacctacacc 540

ttctcccggg cgcccagccc caccaagtcg caggaccaga cgggctcagg ctggaacttc 600

tgctctatcc cctggtgctt gttttgggcc acggtcctgc tgctgatcat ctacctgcag 660

ttctctttcc gtagctccgt ataa 684

<210> 7
<211> 678
<212> DNA
<213> Homo sapiens

<400> 7
atgtgtacca gcttgaccac ttgtgagtgg aagaaagtct tctatgagaa gatggaggtg 60

gcaaagccag cggacagctg ggagctcatc atagacccca acctcaagcc cagtgagctg 120

gcccctggct ggaagcagta cctggagcag cacgcctcag gcaggttcca ctgctcctgg 180

tgctggcaca cctggcagtc tgcccatgtg gtcatcctct tccacatgtt cctggaccgc 240

gcccagcggg cgggctcggt gcgcatgcgc gtcttcaagc agctgtgcta tgagtgcggc 300

acggcgcggc tggacgagtc cagcatgctg gaggagaaca tcgagggcct ggtggacaac 360

ctcatcacca gcctgcgcga gcagtgctac gaggaggatg gtggccagta ccgcatccac 420

gtggccagcc gcccggacag cgggccgcat cgtgcagagt tctgtgaggc ctgccaggag 480

ggcatcgttc actggaagcc cagcgagaag ctgctggagg aggaggtgac cacctacacc 540

tctgaagcct ccaagccgag ggcccaggcg ggatccggct acaacttctt gtctcttcgc 600

tggtgcctct tctgggcctc tctctgcctg ctcgttgttt acctgcagtt ctccttcctc 660

agtcctgcct tcttttag 678

<210> 8
<211> 606
<212> DNA
<213> Homo sapiens

<400> 8
atggtgtcat ggatcatctc caggctggtg gtgcttatat ttggcaccct ttaccctgcg 60

tattattcct acaaggctgt gaaatcaaag gacattaagg aatatgtcaa atggatgatg 120

tactggatta tatttgcact tttcaccaca gcagagacat tcacagacat cttcctttgt 180

tggtttccat tctattatga actaaaaata gcatttgtag cctggctgct gtctccctac 240

acaaaaggct ccagcctcct gtacaggaag tttgtacatc ccacgctatc ttcaaaagaa 300

aaggaaatcg atgattgtct ggtccaagca aaagaccgaa gttacgatgc ccttgtgcac 360

ttcgggaagc ggggcttgaa cgtggccgcc acagcggctg tgatggctgc ttccaaggga 420

cagggtgcct tatcggagag actgcggagc ttcagcatgc aggacctcac caccatcagg 480

ggagacggcg cccctgctcc ctcgggcccc ccaccaccgg ggtctgggcg ggccagcggc 540

aaacacggcc agcctaagat gtccaggagt gcttctgaga gcgctagcag ctcaggcacc 600

gcctag 606

<210> 9
<211> 20
<212> PRT
<213> Homo sapiens

<400> 9

Met Asn Gly Thr Glu Gly Pro Asn Phe Tyr Val Pro Phe Ser Asn Lys
1               5                   10                  15

Thr Gly Val Val
            20

<210> 10

<211>  23
<212>  DNA
<213>  Artificial Sequence

<220>
<223>  primer


<220>
<221>  misc_feature
<222>  (6)..(6)
<223>  n=i

<220>
<221>  misc_feature
<222>  (15)..(15)
<223>  n=i

<220>
<221>  misc_feature
<222>  (21)..(21)
<223>  n=i

<400>  10
atggcntayg aymgntaygt ngc                                              23


<210>  11
<211>  32
<212>  DNA
<213>  Artificial Sequence

<220>
<223>  primer


<220>
<221>  misc_feature
<222>  (3)..(3)
<223>  n is a, c, g, or t

<220>
<221>  misc_feature
<222>  (12)..(12)
<223>  n is a, c, g, or t

<220>
<221>  misc_feature
<222>  (21)..(21)
<223>  n is a, c, g, or t

<220>
<221>  misc_feature
<222>  (27)..(27)
<223>  n is a, c, g, or t

<400>  11
tcncgrttyc tnagrctrta natraanggr tt                                    32


<210>  12
<211>  32
<212>  DNA

<213>  Artificial Sequence

<220>
<223>  primer



<220>
<221>  misc_feature
<222>  (12)..(12)
<223>  n=i

<220>
<221>  misc_feature
<222>  (21)..(21)
<223>  n=i

<220>
<221>  misc_feature
<222>  (24)..(24)
<223>  n=i

<220>
<221>  misc_feature
<222>  (27)..(27)
<223>  n=i

<400>  12
tcyttrttyc tnagrctrta natnatnggr tt                                    32